(19) Europäisches Patentamt
European Patent Office
Office européen des brevets

(11) EP 1 454 986 A1

# (12) EUROPEAN PATENT APPLICATION

(43) Date of publication:
**08.09.2004 Bulletin 2004/37**

(51) Int Cl.[7]: **C12N 15/29**, C12N 15/53, C12N 9/08

(21) Application number: **03290518.4**

(22) Date of filing: **04.03.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(71) Applicant: **CENTRE NATIONAL DE LA RECHERCHE SCIENTIFIQUE (CNRS)
75016 Paris (FR)**

(72) Inventors:
- **Leblanc, Catherine
29640 Plougonven (FR)**
- **Colin, Carole
29680 Roscoff (FR)**
- **Kloareg, Bernard
29420 Plouenan (FR)**
- **Delage, Ludovic
29250 Santec (FR)**
- **Roeder, Vincent, Jean-Michel, Wilfred
29660 Carentec (FR)**
- **Potin, Philippe
29680 Roscoff (FR)**

(74) Representative: **Ahner, Francis et al
Cabinet Régimbeau
20, rue de Chazelles
75847 Paris cedex 17 (FR)**

## (54) Vanadium haloperoxidases from the brown alga Laminaria digitata and uses thereof

(57) The present invention concerns bromo- and iodoperoxidases from *Laminaria digitata,* isolated nucleic acids encoding same, as well as methods for preparing these enzymes.

Moreover, the invention is also directed to the use of such haloperoxidases in a wide range of industrial, pharmaceutical and ecological applications.

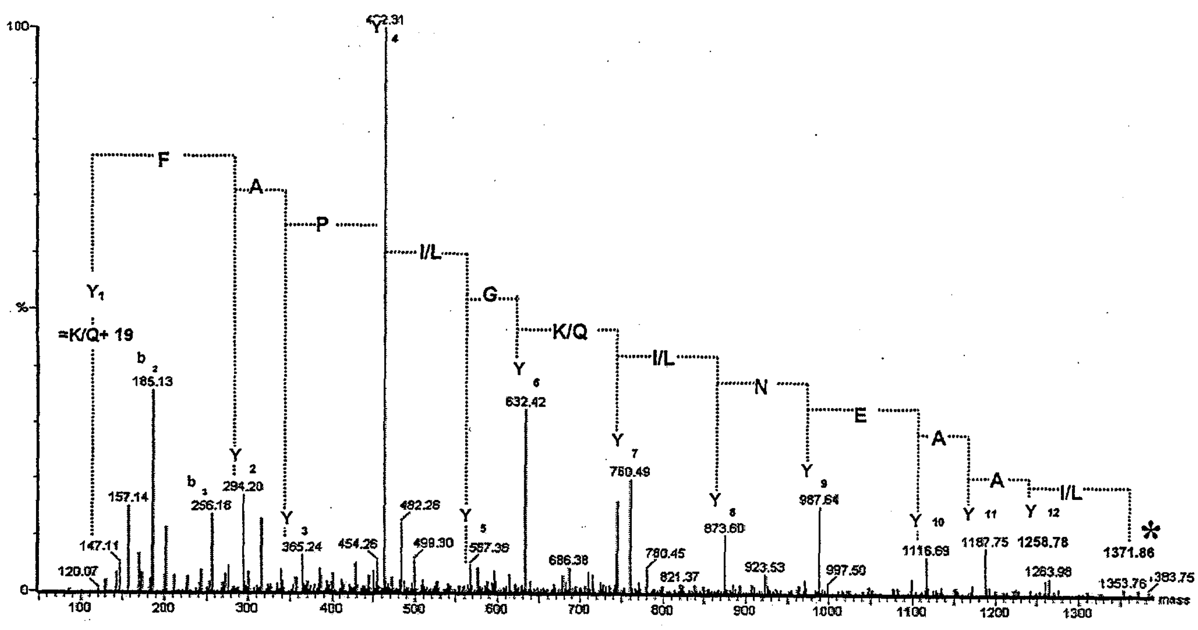


FIGURE 4

EP 1 454 986 A1

SEQUENCE LISTING

<110> CENTRE NATIONAL DE LA RECHERCHE SCIENTIFIQUE - CNRS

<120> HALOPEROXIDASES FROM BROWN ALGAL KELP Laminaria digitata, METHODS OF PREPARATION AND USES THEREOF

<130> D20943

<160> 17

<170> PatentIn version 3.1

<210> 1
<211> 646
<212> PRT
<213> Laminaria digitata

<220>
<223> Bromoperoxidase vBPO1

<400> 1
```
Met Lys Ile Ser Ser Leu Gly Leu Thr Ala Leu Leu Ala Ala Phe Ala
1               5                   10                  15

Pro Cys Leu Gly Tyr Glu Glu Pro Pro Glu Pro Thr Gln Pro Leu Leu
            20                  25                  30

Ser Gly Asn Val Cys Arg Val Arg Asp Ser Leu Asp Phe Leu Asp Pro
        35                  40                  45

Val Pro Arg Ala Lys Val Thr Leu Leu Lys Arg Leu Ala Ile Ala Lys
    50                  55                  60

Asp Glu Ile Ser Val Gly Pro Thr Cys His Ile Asn Asn Gly Asp Glu
65                  70                  75                  80

Glu Asn Val Pro Leu Phe Ala Gly Gln Tyr His Lys Thr Leu Pro His
                85                  90                  95

Asp Lys Phe Gly Gln Val Asp Glu Asp Ala Tyr Lys Lys Leu Leu Glu
            100                 105                 110

Cys Val Phe Thr Ser Asp Ile Asn Glu Cys Glu Lys Val Pro Ser Gly
        115                 120                 125

Ala Gly Arg Arg Gly Gly Ala Lys Leu Thr Asn Pro Leu Gly Gly Thr
    130                 135                 140

Ala His Gln Val Thr Gly Ala Asp Ser Asp Asn Val Phe Ile Thr Thr
145                 150                 155                 160

Pro Asp Ser Leu Leu Ser Glu Arg Leu Ala Ala Gln Gln Ala Glu Val
                165                 170                 175

Tyr Trp Met Ala Leu Leu Arg Asp Ile Pro Phe Gly Glu Phe Ala Lys
            180                 185                 190

Asn Asp Tyr Val Arg Leu Ala Ala Glu Asn Leu Gln Gly Leu Pro Ala
        195                 200                 205
```

```
Phe Lys Gly Leu Asn Ile Pro Arg Ser Glu Gly Gly Lys Ile Asp Pro
    210                 215                 220

Val Thr Asp Leu Phe Arg Thr Thr Trp Pro Gly Val Thr Thr Gly Pro
225                 230                 235                 240

Val Val Ser Gln Phe Met Leu Ser Asp Phe Leu Ile Asp Ser Ile Lys
                245                 250                 255

Val Thr Pro Lys Ala Asp Pro Leu Thr Pro Gly Val Asp Tyr Met Thr
            260                 265                 270

Ala Phe Gln Pro Trp Leu Asp Val Gln Asn Gly Ala Ser Lys Leu Glu
        275                 280                 285

Thr Thr Phe Asp Glu Glu Asn Pro Arg Phe Ile Arg Asn Gly Arg Asp
    290                 295                 300

Leu Ala Thr Ile Ala Leu Arg Asp Gln Leu Tyr Thr Glu Ala Phe Arg
305                 310                 315                 320

Ala Ala Leu Ile Leu Phe Thr Glu Gly Ala Leu Gly Gly Glu Val Gly
                325                 330                 335

Pro Tyr Ala Glu Ala Glu Arg Gln Gln Gly Phe Ala Thr Phe Gly Glu
            340                 345                 350

Pro His Ile Leu Thr Ala Met Ala Ser Ala Ser Ser Val Thr Arg His
        355                 360                 365

Ala Trp Tyr Ala Lys Trp Gln Val His Arg Met Leu Arg Pro Glu Ala
    370                 375                 380

Tyr Gly Ala Leu Val His Asn Thr Leu Met Arg Asp Val Ile Thr Pro
385                 390                 395                 400

Leu Pro Asp Ser Ile Leu Arg Asn Thr Glu Leu Leu Asn Arg Val Glu
                405                 410                 415

Val His Asn Gln Arg Met Asn Pro Asp Gly Glu Lys Thr Phe Leu Leu
            420                 425                 430

Pro Met Ala Ala Ala Gln Gly Ser Pro Thr His Pro Ala Tyr Pro Ser
        435                 440                 445

Gly His Ala Ile Asn Asn Gly Ala Tyr Ile Thr Ala Leu Lys Ala Phe
    450                 455                 460

Leu Gly Tyr Glu Ala Gly Gln Lys Cys Phe Pro Asn Pro Val Val Ser
465                 470                 475                 480

Asn Asp Glu Gly Thr Lys Arg Ile Lys Tyr Lys Pro Ser Gly Arg Glu
                485                 490                 495

Ile Val Gly Glu Cys Val Asn Glu Lys Gly Lys Leu Val Glu Gly Leu
            500                 505                 510

Thr Tyr Glu Gly Glu Leu Asn Lys Ile Ser Ala Asn Val Leu Leu Gly
        515                 520                 525

Arg Ser His Ile Gly Val His Trp Arg Met Asp Gly Val Tyr Gly Ala
```

```
    530                 535                 540

Leu Met Gly Glu Thr Ser Cys Val Arg Arg Leu Gln Gln Glu Leu Pro
545                 550                 555                 560

Gly Leu Pro Glu Ala Arg Glu Val Glu Gly Lys Lys Arg Arg Gly Asp
                565                 570                 575

Ile Pro Pro Ala Thr Tyr Lys Phe Arg Leu Tyr Ser Gly Lys Ile Leu
            580                 585                 590

Glu Leu Tyr Gly Arg Asn Leu Tyr Lys Leu Asp Gly Lys Leu Cys Glu
        595                 600                 605

Gly Ala Phe Thr Gly Asp Asp Phe Cys Asp Pro Ile Asp Glu Asp Asp
    610                 615                 620

Tyr Ser Ser Phe Asp Asp Ile Val Glu Glu His Ala Gln Phe Ser Leu
625                 630                 635                 640

His Gly His Thr Glu Leu
                645


<210> 2
<211> 682
<212> PRT
<213> Laminaria digitata

<220>
<223> Bromoperoxidase vBPO2

<400> 2
Met Lys Arg Ala Arg Pro Thr Gln Pro Gly Ala Leu Tyr Ser Val Leu
1               5                   10                  15

Ser Leu Arg His Val Ala Cys Ala Leu Cys Val Val Ala Cys Ala Phe
            20                  25                  30

Leu Leu Phe Glu Tyr Gln Ile Ser Ser Leu Gly Leu Thr Ala Leu Leu
        35                  40                  45

Ala Ala Phe Ala Pro Cys Leu Gly Tyr Glu Glu Pro Pro Glu Pro Thr
    50                  55                  60

Gln Pro Leu Leu Ser Gly Asn Val Cys Arg Val Arg Asp Ser Leu Asp
65                  70                  75                  80

Phe Leu Asp Pro Val Pro Arg Ala Lys Val Thr Leu Leu Lys Arg Leu
                85                  90                  95

Ala Ile Ala Lys Asp Glu Ile Ser Val Gly Pro Thr Cys His Val Asn
            100                 105                 110

Asn Gly Asp Glu Glu Asn Val Pro Leu Phe Ala Gly Gln Tyr His Lys
        115                 120                 125

Thr Leu Pro His Asp Lys Phe Gly Gln Val Asp Glu Asp Ala Tyr Lys
    130                 135                 140

Lys Leu Leu Glu Cys Val Phe Thr Ser Asp Ile Asn Glu Cys Glu Lys
```

145 150 155 160

Val Pro Ser Gly Ala Gly Arg Arg Gly Gly Ala Lys Leu Thr Asn Pro
165 170 175

Leu Gly Gly Thr Ala His Gln Val Thr Gly Ala Asp Ser Asp Asn Val
180 185 190

Phe Ile Thr Thr Pro Asp Ser Leu Leu Ser Glu Arg Leu Ala Ala Gln
195 200 205

Gln Ala Glu Val Tyr Trp Met Ala Leu Leu Arg Asp Ile Pro Phe Gly
210 215 220

Glu Phe Ala Lys Asn Asp Tyr Val Arg Leu Ala Ala Glu Asn Leu Gln
225 230 235 240

Ser Leu Pro Ala Phe Lys Gly Leu Asn Ile Pro Arg Ser Glu Gly Gly
245 250 255

Lys Ile Asp Pro Val Thr Asp Leu Phe Arg Thr Thr Trp Pro Gly Val
260 265 270

Thr Thr Gly Pro Val Val Ser Gln Phe Met Leu Ser Asp Phe Leu Ile
275 280 285

Asp Ser Ile Lys Val Thr Pro Lys Ala Asp Pro Leu Thr Pro Gly Val
290 295 300

Asp Tyr Met Thr Ala Phe Gln Pro Trp Leu Asp Val Gln Asn Gly Ala
305 310 315 320

Ser Lys Leu Glu Thr Thr Phe Asp Glu Glu Asn Pro Arg Phe Ile Arg
325 330 335

Asn Gly Arg Asp Leu Ala Thr Ile Ala Leu Arg Asp Gln Leu Tyr Thr
340 345 350

Glu Ala Phe Arg Ala Ala Leu Ile Leu Phe Thr Glu Gly Ala Leu Gly
355 360 365

Gly Glu Val Gly Pro Tyr Ala Glu Ala Glu Arg Gln Gln Gly Phe Ala
370 375 380

Thr Phe Gly Glu Pro His Ile Leu Thr Ala Met Ala Ser Ala Ser Ser
385 390 395 400

Ser Thr Arg His Ala Trp Tyr Ala Lys Trp Gln Val His Arg Met Leu
405 410 415

Arg Pro Glu Ala Tyr Gly Ala Leu Val His Asn Thr Leu Met Arg Asp
420 425 430

Val Ile Thr Pro Leu Pro Asp Ser Ile Leu Arg Asn Thr Glu Leu Leu
435 440 445

Asn Arg Val Glu Val His Asn Gln Arg Met Asn Pro Asp Gly Glu Lys
450 455 460

Thr Phe Leu Leu Pro Met Ala Ala Ala Gln Gly Ser Pro Thr His Pro
465 470 475 480

```
Ala Tyr Pro Ser Gly His Ala Ile Asn Asn Gly Ala Tyr Ile Thr Ala
                485                 490                 495

Leu Lys Ala Phe Leu Gly Tyr Glu Ala Gly Gln Lys Cys Phe Pro Asn
            500                 505                 510

Pro Val Val Ser Asn Asp Glu Gly Thr Lys Arg Ile Lys Tyr Lys Pro
        515                 520                 525

Ser Gly Arg Glu Ile Val Gly Glu Cys Val Asn Glu Lys Gly Lys Leu
    530                 535                 540

Val Glu Gly Leu Thr Tyr Glu Gly Glu Leu Asn Lys Ile Ser Ala Asn
545                 550                 555                 560

Val Leu Leu Gly Arg Ser His Ile Gly Val His Trp Arg Met Asp Gly
                565                 570                 575

Val Tyr Gly Ala Leu Met Gly Glu Thr Ser Cys Val Arg Arg Leu Gln
            580                 585                 590

Gln Glu Leu Pro Gly Leu Pro Glu Ala Arg Glu Val Glu Gly Lys Lys
        595                 600                 605

Arg Arg Gly Asp Ile Pro Pro Ala Thr Tyr Lys Phe Arg Leu Tyr Ser
    610                 615                 620

Gly Lys Ile Leu Glu Leu Tyr Gly Arg Asn Leu Tyr Lys Leu Asp Gly
625                 630                 635                 640

Lys Leu Cys Glu Gly Ala Phe Thr Gly Asp Asp Phe Cys Asp Pro Ile
                645                 650                 655

Asp Glu Asp Asp Tyr Ser Ser Phe Asp Asp Ile Val Glu Glu His Ala
            660                 665                 670

Gln Phe Ser Leu His Gly His Thr Glu Leu
        675                 680


<210> 3
<211> 625
<212> PRT
<213> Laminaria digitata

<220>
<223> iodoperoxidase vIPO

<400> 3
Ile Met Lys Gly Leu Ala Gly Pro Ala Gly Ala Met Ala Val Val Ala
1               5                   10                  15

Leu Gly Leu Val Pro Gly Gly Ala Ile Gly Lys Ser Leu Arg Gln Glu
            20                  25                  30

Pro Ser Glu Pro Arg Leu Ser Gly Gly Val Asp Thr Ala Ala Ser Pro
        35                  40                  45

Ser Lys Asp Thr Leu Lys Gly Ser Leu Ser Arg Lys Leu Gln Val Val
    50                  55                  60
```

```
Asn Asp Asp Ala Leu Asp Val Ser Gly Thr Pro Ala Glu Arg Ala Ala
65                  70                  75                  80

Asn Ala Leu Asn Gln Arg Ile Glu Phe Ala Glu Thr Glu Phe Thr Ala
                85                  90                  95

Ser Glu Gly Thr Leu His Leu Asn Asn Gly Asp Arg Ser Ser Ala Ala
            100                 105                 110

Thr Phe His Lys Ser Leu Pro His Asp Ser Leu Gly Gln Val Asn Ser
        115                 120                 125

Glu Asp Phe Asp Leu Leu Met Glu Cys Ile Ala Gln Gly Asp Phe Asp
    130                 135                 140

Thr Cys Glu Leu Val Pro Ala Gly Asp Asp Gly Arg Leu Ser Asn Pro
145                 150                 155                 160

Leu Gly Gly Ile Ala Val Glu Met Ala Gly Ala Ala Gly Pro Ala Leu
                165                 170                 175

Thr Leu Pro Pro Ala Ser Ala Ile Asn Ser Glu Asp Leu Ala Ala Gln
            180                 185                 190

Met Ala Glu Gln Tyr Trp Met Ala Leu Thr Arg Asp Val Pro Phe Ser
        195                 200                 205

Gln Tyr Gly Glu Asp Glu Ala Thr Val Ala Ala Ala Asp Asn Leu Ala
    210                 215                 220

Thr Met Pro Gly Phe Ala Asp Ile Val Gly Val Ala Val Asp Pro Glu
225                 230                 235                 240

Thr Arg Arg Ala Asp Pro Gln Ser Gln Leu Phe Arg Ser Ser Ala Phe
                245                 250                 255

Gly Val Glu Thr Gly Pro Phe Ile Ser Gln Leu Leu Val Lys Asp Phe
            260                 265                 270

Thr Ile Asp Ser Ile Thr Val Thr Pro Met Gln Lys Thr Phe Ala Pro
        275                 280                 285

Gly Ala Asp Tyr Met Thr Asp Tyr Asp Glu Trp Leu Ser Ile Gln Asn
    290                 295                 300

Gly Gly Ser Pro Asp Ser Glu Ala Asp Leu Asp Asp Glu Asp Arg Tyr
305                 310                 315                 320

Ile Arg Asn Ser Arg Asp Leu Ser Arg Leu Val Ala Thr Asp Thr Val
                325                 330                 335

Asn Thr Glu Ala Tyr Arg Ala Ala Leu Ile Leu Leu Asp Pro Asp Gln
            340                 345                 350

Gly Ala Asp Gly Arg Ala Ala Ile Ser Ala Pro Gly Leu Asn Gly Pro
        355                 360                 365

Tyr Ala Asp Ser Ser Arg Gln Ala Gly Phe Val Asn Tyr Gly Val Ser
    370                 375                 380
```

```
His Leu Met Arg Leu Val Gly Thr Ala Glu Leu Ala Gln Lys Ser Ala
385                 390                 395                 400

Trp Tyr Gln Lys Trp Asn Val His Met Phe Val Arg Pro Glu Ala Phe
                405                 410                 415

Gly Gly Ser Ile His Asn Val Leu Leu Gly Lys Leu Asp Val Glu Ile
            420                 425                 430

Ala Pro Ser Leu Leu Lys Asn Thr Asp Leu Leu Asp Arg Val Ala Ala
        435                 440                 445

Arg Asn Gly Glu Ile Asn Gly Arg Pro Gly Val Leu Asp Arg Thr Tyr
    450                 455                 460

Leu Leu Ser Gln Ala Leu Pro Glu Gly Ser Pro Thr His Pro Ser Tyr
465                 470                 475                 480

Pro Ala Gly His Ala Thr Gln Asn Gly Ala Phe Ala Thr Val Leu Lys
                485                 490                 495

Ala Leu Val Gly Leu Glu Arg Gly Ser Val Cys Phe Asn Asp Pro Val
            500                 505                 510

Phe Pro Asp Asp Glu Gly Leu Thr Leu Leu Pro Tyr Thr Gly Asp Asp
        515                 520                 525

Gly Asn Asn Cys Leu Thr Phe Glu Gly Glu Ile Asn Lys Leu Ala Val
    530                 535                 540

Asn Val Ala Leu Gly Arg Asn Met Leu Gly Val His Trp Arg Ile Asp
545                 550                 555                 560

Ser Glu Leu Gly Leu Leu Leu Gly Glu Thr Ala Ala Val Arg Ile Leu
                565                 570                 575

Gln Gln Glu Ala Val Ala Tyr Pro Glu Asn Ala Gly Tyr Glu Phe Arg
            580                 585                 590

Leu Met Ser Gly Lys Thr Ile Arg Leu Glu Thr Asp Gly Thr Phe Phe
        595                 600                 605

Ile Asp Asp Thr Leu Cys Ser Gly Asp Ala Phe Met Gly Ala Asp Leu
    610                 615                 620

Cys
625
```

<210> 4
<211> 3379
<212> DNA
<213> Laminaria digitata

<220>
<223> Sequence coding for a bromoperoxidase vBPO1

<400> 4

```
ccgaaccgaa caagctttcc tcttcggccc ctaacacttc gaggcgggcc aaaggcacct      60
acggcggccg gctgcatacc aagagtttag acacaagcct cgccatgaag atttcttcct     120
tgggcttgac cgctttgctg gcggcgttcg ctccctgcct cggctacgag gagcctcccg     180
```

```
agcccacgca gcctctgctc agcgggaacg tgtgcagggt gcgagactcc ctcgacttcc    240
ttgacccggt gccgcgcgcg aaggtgaccc tgctcaagcg cctggccatc gccaaggacg    300
agatatcggt tggacccact tgccacatca acaacggcga cgaggaaaac gtcccgctct    360
tcgctgggca gtaccacaag acgctaccgc acgacaagtt cggtcaggtg gacgaagacg    420
catacaagaa gctgctggag tgcgtcttca cgagcgacat caacgagtgc gagaaggtgc    480
cctccggcgc cggccgaaga ggtggcgcga agctgaccaa ccccctcggg ggcaccgcgc    540
accaggtcac cggtgctgac agcgacaatg tgttcatcac cacgcccgac agtctcctct    600
ccgaaaggct ggccgcgcag caggcagaag tttactggat ggccctgctt agggacatcc    660
ccttcgggga gttcgccaag aacgactacg tcaggctcgc cgcagagaac ctgcagggcc    720
tgccggcgtt caagggactc aacattcccc ggagcgaggg aggaaagatc gaccctgtca    780
ctgatctgtt ccgaacgacc tggccgggtg tgaccaccgg tcccgtcgtg tctcagttca    840
tgctgtcgga cttcctgatc gactctatca aggtcacccc gaaggctgac ccccttaccc    900
caggggtgga ctacatgacc gccttccagc cgtggctcga cgtacagaac ggggcttcca    960
agcttgaaac tactttcgac gaggagaacc cccgcttcat ccgcaacggc cgagacctgg   1020
ccactatcgc cttgagggat caactctaca cggaggcctt ccgcgccgcg ctcatcctgt   1080
tcacggaagg tgcacttggc ggcgaggtcg ggccttacgc ggaggccgag cgccagcagg   1140
ggttcgccac tttcggggag ccgcacatcc tcaccgccat ggcctcggcc agctctgtga   1200
cgcgccacgc gtggtacgct aagtggcaag tccaccgcat gctgcgaccc gaggcgtacg   1260
gtgccctggt acataacacg ctcatgaggg acgtcatcac cccgctgccg gactctatcc   1320
tgaggaacac ggagctcctc aaccgagtgg aggtgcacaa ccagcgtatg aacccagacg   1380
gcgagaagac cttcctgctc cccatggcgg cagcccaggg ctctcccacg caccccgctt   1440
accccagtgg acacgccatc aacaacggcg cctacatcac cgcgctcaag gcgttcctcg   1500
ggtacgaggc cggccagaag tgcttcccca accccgtggt ctcgaacgac gagggaacca   1560
agaggatcaa atacaagccc tccggaaggg agattgtggg cgagtgcgtc aacgagaagg   1620
gcaagctcgt cgaaggcctc acgtacgagg gagagctcaa caagatcagc gccaacgtgc   1680
tcttgggaag gtcccacatc ggtgttcact ggaggatgga cggcgtgtac ggagcgctca   1740
tgggagagac cagctgcgtc cgccgcctgc agcaggaact tcctggcctc ccggaggccc   1800
gcgaggttga gggcaagaag cgccgtggtg acatcccccc ggcgacttac aaattccgcc   1860
tttacagcgg taagattctt gagctctacg gcaggaactt gtacaagctg gacggcaagc   1920
tctgcgaggg ggccttcacc ggcgacgact tctgtgaccc gatcgacgag gacgactaca   1980
gctcgttcga cgacatcgtc gaggaacacg cccagttctc gctccacgga cacaccgagc   2040
tctaaataac gtcacagact caagacaccc atcagtcgca gtttcggtaa tcgcgtattt   2100
cgcaaatccg atagccgcgg atttcgcgca ttcggtagcc gcggatttcg tggccacgga   2160
ttcggtggtc acggattcgg tgggtacgac gtccaggaat acgcgcactt ctcgctccac   2220
accgagctct aagctacgtc acggactgaa gacacccatc atggacacac tgaggagaga   2280
ccggaataat ccggtgatgc gtcgggcgcg aagtacttcg gtcactgtta accccttag    2340
gactgcacta ttcctatttc gaggacgaag tgcttggaat tacactaaga tatgtgcttc   2400
tgcacagtgc agcggaaaca aaatggttga gacgccttgg tcgcttgaaa gggctgctct   2460
gccgtatgcg agaaaccatg tggtacgtaa ggtggtgggt tcgattcggt tcttttaggt   2520
agtgccgtgc gtgctcttta cagggtacga aggatttgtt tggttgtttt ggaatgatca   2580
cgggagatgc tatgctttgt aagataaagg ggcaggctaa actgcttgtc aacactattc   2640
tgaggctgga tggagaggga gagacatatc gttgcgatct ctctgtacgc atgtaggtgc   2700
tgcgacagtg ccgtagggta taggaggaag agaaatacac ttttcgtgtt gagggcgtgg   2760
ttcggctact gttgctatag agtatatact tcgtattatg tatgcggtgt gaacaccgat   2820
actctacgta ctatttatca gttcattgca atatatgtct tctgatttat cgaaaagaag   2880
actactctga tgcaagggac tacgtacagt acgaggtgga cggattggcg tttataagcg   2940
tggcgtaagg cggtgctcgt ttggatttgc acgattggca tcttcgaagg aaggaaaact   3000
gttgcatatc caatctggct ttgctacgcc ctgatttcaa tgcggcggct agacgtgata   3060
cgtcgacatt ttccgatgtc aagttcaggg ctagggttga gcggaacagt cagagaggt    3120
aatgctgctc aggttatatt gattatgtga taattccgag cgcgacgagg agtcggcgag   3180
taagaaacat attccattta gtcgttgctt tcccaggtgt gcgaatcatt cgggttatca   3240
acgatttggt tcgtaggaca agtacaggta gtaacgtcta gcttgttgaa cgtacagcgg   3300
agttatcgcg acgaatgcaa ctccgaatca acgtttggtg ttttggtaca caactaatat   3360
tataccctat tggcgtttc                                                3379
```

<210> 5
<211> 3420
<212> DNA
<213> Laminaria digitata

<220>
<223> Sequence coding for a bromoperoxidase vBPO2

<400> 5
```
cggctgcata ccaagagttt agacacaagc ctcgccatga agcgcgctag gcccacccag      60
cccggcgcgc tgtactctgt cctctctctc cgacacgtgg cgtgtgccct gtgcgttgtc     120
gcatgcgctt tccttttgtt cgaatatcag atttcttcct tgggcttgac cgctttgctg     180
gcggcgttcg ctccctgcct cggctacgag gagcctcccg agcccacgca gcctctgctc     240
agcgggaacg tgtgcagggt gcgagactcc ctcgacttcc ttgacccggt gccgcgcgcg     300
aaggtgaccc tgctcaagcg cctggccatc gccaaggacg agatatcggt tggacccact     360
tgccacgtca acaacggcga cgaggaaaac gtcccgctct tcgctgggca gtaccacaag     420
acgctaccgc acgacaagtt cggtcaggtg gacgaagacg catacaagaa gctgctggag     480
tgcgtcttca cgagcgacat caacgagtgc gagaaggtgc cctccggcgc cggccgaaga     540
ggtggcgcga agctgaccaa ccccctcggg ggcaccgcgc accaggtcac cggtgctgac     600
agcgacaatg tgttcatcac cacgcccgac agtctcctct ccgaaaggct ggccgcgcag     660
caggcagaag tttactggat ggccctgctt agggacatcc ccttcgggga gttcgccaag     720
aacgactacg tcaggctcgc cgcagagaac ctgcaaagcc tgccggcgtt caagggactc     780
aacattcccc ggagcgaggg aggaaagatc gaccctgtca ctgatctgtt ccgaacgacc     840
tggccgggtg tgaccaccgg tcccgtcgtg tctcagttca tgctgtcgga cttcctgatc     900
gactctatca aggtcacccc gaaggctgac cccccttaccc caggggtgga ctacatgacc     960
gccttccagc cgtggctcga cgtacagaac ggggcttcca agcttgaaac tactttcgac    1020
gaggagaacc cccgcttcat ccgcaacggc cgagacctgg ccactatcgc cttgagggat    1080
caactctaca cggaggcctt ccgcgccgcg ctcatcctgt tcacggaagg tgcacttggc    1140
ggcgaggtcg ggccttacgc ggaggccgag cgccagcagg ggttcgccac tttcggggag    1200
ccgcacatcc tcaccgccat ggcctcggcc agctcttcca cgcgccacgc gtggtacgct    1260
aagtggcaag tccaccgcat gctgcgaccc gaggcgtacg gtgccctggt acataacacg    1320
ctcatgaggg acgtcatcac cccgctgccg gactctatcc tgaggaacac ggagctcctc    1380
aaccgagtgg aggtgcacaa ccagcgtatg aacccagacg gcgagaagac cttcctgctc    1440
cccatggcgg cagcccaggg ctctcccacg caccccgctt accccagtgg acacgccatc    1500
aacaacggcg cctacatcac cgcgctcaag gcgttcctcg ggtacgaggc cggccagaag    1560
tgcttcccca accccgtggt ctcgaacgac gagggaacca agaggatcaa atacaagccc    1620
tccggaaggg agattgtggg cgagtgcgtc aacgagaagg gcaagctcgt cgaaggcctc    1680
acgtacgagg gagagctcaa caagatcagc gccaacgtgc tcttgggaag gtcccacatc    1740
ggtgttcact ggaggatgga cggcgtgtac ggagcgctca tgggagagac cagctgcgtc    1800
cgccgcctgc agcaggaact tcctggcctc ccggaggccc gcgaggttga gggcaagaag    1860
cgccgtggtg acatcccccc ggcgacttac aaattccgcc tttacagcgg taagattctt    1920
gagctctacg gcaggaactt gtacaagctg gacggcaagc tctgcgaggg ggccttcacc    1980
ggcgacgact tctgtgaccc gatcgacgag gacgactaca gctcgttcga cgacatcgtc    2040
gaggaacacg cccagttttc gctccacgga cacaccgagc tctaaataac gtcacagact    2100
caagacaccc atcagtcgca gtttcggtag tcgcgtattt cgcaaatccg atagccgcgg    2160
atttcgcgca ttcggtagcc gcggatttcg tggccacgga ttcggtggtc acggattcgg    2220
tgggtacgac gtccaggaat acgcgcactt ctcgctccac accgagctct aagctacgtc    2280
acggactgaa gacacccatc atggagagac tgaggagaga ccggaataat ccggtgatgc    2340
gtcgggcgcg aagtacttcg gtcactgtta accccttttag gactgcacta ttcctatttt    2400
gaggacgaag tgcttggaat tatactaaga taagtgcttc tgcacagtgc agcggaaaca    2460
aaatggttga gacgccttgg tcgcttgaaa gggctgctct gccgtatgcg agaaaccatg    2520
tggtacgtaa ggtggtgggt tcgattcggt tcttttaagt agtgccgtgc gtgctcttta    2580
cagggtacga aggatttgtt tggttgtttt ggaatgatca tgggagatgc tatgctttgt    2640
aagataaagg ggcaggctaa actgcttgtc ccaactattc tgaggctgga tggagaggga    2700
gagacatatc gttgcgatct ctctgtacgc atgtaggtgc tgcgacagtg ccgtagggta    2760
tagcaggaag agaaatacac ttttcgtgtt gagggcgtgg ttcggctact gttgctatag    2820
agtatatact tcgtattatg tatgcggtgt gaacaccgat actctacgta ctatttatca    2880
attcattgca atatatgtct tctgatttat cgaaatgaag actactctga tgcaagggac    2940
tacgtacagt acgaggtgga cggattggtg tttataagcg tggcgtaagg cggtgctcgt    3000
ttggattttc acgattggca tcttcgaagg aaggaaaact gttgcatatc caatctggct    3060
ttgctacgcc ctgatttcaa tgccgcggct agacgtgata cgtcgacatt aatccgatgt    3120
caagttcagg gctagggttg agcggaacag tcagagaggg taatgctgct caggttatat    3180
tgattatgtg ataattccga gcgcgacgag gagtcggcga gtaagaaaca tattccattt    3240
agtcgttgct ttcccaggtg tgcgaatcat tcgggttatc aacgatttgg ttcgtaggac    3300
aagtacaggt agtaacgtct ggcttgttga acgtacagcg gagttatcgc gacgaatgca    3360
```

actctgaatc aacgtttggt gttttggtac acaactaata ttataccta ttggcgtttc 3420

<210> 6
<211> 3730
<212> DNA
<213> Laminaria digitata

<220>
<223> Sequence coding for a iodoperoxidase vIPO

<400> 6
agaaaaagca ccgcctactg ctgccacgcc agaggaggac accatacact gtacttcctg 60
ttctgcatgc tgtaccgcgc ataggaatat catctgacca gcgtgcccat aaaaaaaggt 120
cgacatcgac tcgcaacatg aaggggcttg caggaccagc cggtgctatg gccgttgtcg 180
cgctcgggct tgtccccggc ggagcaatcg ggaaatcttt gcgacaagag ccctctgaac 240
cccgcctaag tggcggcgtg gatacggctg catcgccatc gaaggacacc ctgaaaggga 300
gtctttcgcg taagctccaa gtcgtcaacg atgatgccct cgatgttagt ggcacgcccg 360
cagaaagagc tgccaacgcg ctgaaccagc ggatcgaatt tgcggagacg gagttcacgg 420
catccgaagg cacgctccac cttaacaacg gagaccgctc atccgccgcc acgttccaca 480
agtcgctgcc gcacgacagc ctaggacagg tgaacagcga ggactttgac ctcctcatgg 540
agtgcatcgc tcaaggtgat ttcgacacat gcgagctggt gccggccgga gacgacggca 600
ggctgtccaa ccccctcggg ggtatcgccg tcgagatggc gggagccgcc ggccccgctt 660
tgaccctccc tccggcctca gcgatcaact ccgaggactt ggctgctcaa atggcggaac 720
agtactggat ggccttgacc agggacgtac ctttctctca gtacggcgag gatgaggcga 780
cagtggctgc agcagacaac ttggccacca tgcctggttt tgccgacatt gtcggggtgg 840
ccgtcgatcc ggaaaccaga agagcggatc cgcagtcgca gcttttccgg tcctctgcct 900
tcggcgtcga gacagggccc ttcatttccc agctactggt gaaggacttc acgattgatt 960
ctatcactgt gacgcctatg cagaagacgt ttgcgcccgg agcagactac atgaccgact 1020
acgacgaatg gctctccata cagaacggtg gcagccctga ctcggaagcg gacctagacg 1080
acgaggaccg gtacatccgc aactcccgcg acctctctag gctggtggct accgacaccg 1140
tcaacacgga ggcgtaccga gccgctctga ttcttctcga cccggaccag ggagccgatg 1200
gccgggcagc catcagcgct cccggcttga acggtcccta cgcggacagc agccgccagg 1260
ccggcttcgt caactacggc gtgtctcacc ttatgaggct cgtcggaacc gccgagctgg 1320
cccagaagtc cgcgtggtac caaaagtgga acgtgcacat gttcgtacgt ccggaggctt 1380
tcggcggaag catccacaac gtcctcttgg gcaaactcga cgtagagatc gccccctcgc 1440
ttctcaaaaa cacggatttg ctagacaggg tggctgcacg gaacggggaa atcaacgggc 1500
ggccaggagt actcgaccgc acctacctcc tctcccaggc cctcccccgag gggtcgccaa 1560
ctcacccatc gtaccccgct gggcacgcca cccagaacgg tgcattcgcc acggtgctca 1620
aggccctggt cgggctggag cgtggctctg tctgcttcaa tgaccccgtg ttccccgacg 1680
acgaagggct gacccttctg ccctacaccg gagacgacgg aaacaactgc ctaacattcg 1740
agggagaaat caacaagctg gccgtcaacg tggcattggg caggaacatg ttgggtgttc 1800
actggaggat cgacagcgag ttgggtttgc tcctcggcga gacggcagct gtgaggatcc 1860
tgcagcagga ggccgtggca tacccagaga acgcgggata cgagttccgt ctgatgtcag 1920
gcaagaccat caggctcgaa accgacggga cattcttcat cgacgacacg ctgtgcagcg 1980
gggacgcgtt catgggagct gacctgtgct aatacgtcac acaagtgcca aggatctcgc 2040
tgctccacta gaaccgttta tgcggcggac gtggcccgat cgtaaaggca gtatcatatc 2100
gattgaacgt gcgctgggtt tcagcggcta ctactatgtc aacagtaaac ttgtaaaatt 2160
ggtcttcata aacagggcag tgttagatcg acgactggaa acgccagagg attgagatca 2220
cccaggtatt gctgaaaaag agtttcgcag cagtgatctc ttttgttacg gctatttcac 2280
atttgataca tacatcgccc gtttcaagac ctacgctgta caaacaacta tagctagaag 2340
tgttcagcgg gcaacgtcgg caaagtatcg gccttcagcg gtggacggct gtggggactc 2400
tgtttggacc acacgtttat taatctgttg acgtggaagg gaaagcctgc gtatccctaa 2460
ggggcgcagc gggggcggtg atggttggag tatcgatgct aagcgtccta tttatttgtg 2520
caagtaagtg gttgttgttt tggtaaatac ggtggactcg agcgcgaggt atgtgctggg 2580
gagcaagcca ccgtctacgg catgtttgag acacccaccc tgggcggcag cagaggtcgt 2640
aaggtcccag acgtgtcaaa cgggttgggt tttaagcgaa tcgctgttag gacggcatgc 2700
ggtagcttcg gcgggcctat ctctgcccgc cgcagagtac ggcgtgatcg aattggctcg 2760
ttctcgggtg gcgagcatgg ctatctactg tagagttagg gttaggagca tgaatggtac 2820
attacactgg tgttttgttg tatgtgttaa gaacaacagt ctcagtacct gtatcactac 2880
taaccacgtc ggtgtctgga aatattgcct tccaactttc cttctgctac aagatattta 2940

attttaactg taggcatatg cacacttggc catttgtaag tgcgtgtttc gtcttatgtg 3000
taggtgtaga aggtacatct cttcattaac atttgtaatt ataaccgtac acacagcaca 3060
gccagcttct tgctggtctt ttcacgcgaa gtcgcaaaac agtttcgtcg atattccgtc 3120
gttcatggtc gtggaaagat tgcctgtaag tactttgcat ttcttccttc ttgattcatg 3180
cacggccgtg catgacgcta tcgtttgctt cccttggaaa atttggcgtg ttatccgaag 3240
tagatcagcc ataatagtct atacatgtag ccctttttcga ttgaatcgac gacccggtct 3300
tttgtgtgta tgagtgcctc ttggccgcgg ttttgagctc ctcaagtgag gaactaccgg 3360
tttgtgtgag aatttcacta tgaatagtag aattagtttt gggactatta atagcgcata 3420
taaatgttgg tctgttcgtg cgatcgaaat ttgctcgaag tcatgtttcg gggggaaattt 3480
tgagctcctc aagtgaggag ctcagaacat tctgctgcac ccctataccc ttgagattac 3540
tctcgataaa tcacagtagt ctcgtggttt tttcgagttt ccaacaaatt ggtttcgcag 3600
tgcgactagt ctcgcggttt tttttatttt ccaacaaatt ggtttcgcag ggcgacaccg 3660
ttgaaggttc tgccaaacac gtttgtttac cgtcatcggc aattaaataa taacaacggg 3720
gttgatcctg 3730

<210> 7
<211> 1938
<212> DNA
<213> Laminaria digitata

<220>
<223> ORF coding for a bromoperoxidase vBPO1

<400> 7
atgaagattt cttccttggg cttgaccgct ttgctggcgg cgttcgctcc ctgcctcggc 60
tacgaggagc ctcccgagcc cacgcagcct ctgctcagcg ggaacgtgtg cagggtgcga 120
gactccctcg acttccttga cccggtgccg cgcgcgaagg tgaccctgct caagcgcctg 180
gccatcgcca aggacgagat atcggttgga cccacttgcc acatcaacaa cggcgacgag 240
gaaaacgtcc cgctcttcgc tgggcagtac cacaagacgc taccgcacga caagttcggt 300
caggtggacg aagacgcata caagaagctg ctggagtgcg tcttcacgag cgacatcaac 360
gagtgcgaga aggtgccctc cggcgccggc cgaagaggtg gcgcgaagct gaccaacccc 420
ctcgggggca ccgcgcacca ggtcaccggt gctgacagcg acaatgtgtt catcaccacg 480
cccgacagtc tcctctccga aaggctggcc gcgcagcagg cagaagttta ctggatggcc 540
ctgcttaggg acatcccctt cggggagttc gccaagaacg actacgtcag gctcgccgca 600
gagaacctgc agggcctgcc ggcgttcaag ggactcaaca ttccccggag cgagggagga 660
aagatcgacc ctgtcactga tctgttccga acgacctggc cgggtgtgac caccggtccc 720
gtcgtgtctc agttcatgct gtcggacttc ctgatcgact ctatcaaggt caccccgaag 780
gctgaccccc ttaccccagg ggtggactac atgaccgcct tccagccgtg gctcgacgta 840
cagaacgggg cttccaagct tgaaactact ttcgacgagg agaacccccg cttcatccgc 900
aacggccgag acctggccac tatcgccttg agggatcaac tctacacgga ggccttccgc 960
gccgcgctca tcctgttcac ggaaggtgca cttggcggcg aggtcgggcc ttacgcggag 1020
gccgagcgcc agcaggggtt cgccactttc ggggagccgc acatcctcac cgccatggcc 1080
tcggccagct ctgtgacgcg ccacgcgtgg tacgctaagt ggcaagtcca ccgcatgctg 1140
cgacccgagg cgtacggtgc cctggtacat aacacgctca tgagggacgt catcaccccg 1200
ctgccggact ctatcctgag gaacacggag ctcctcaacc gagtggaggt gcacaaccag 1260
cgtatgaacc cagacggcga gaagaccttc ctgctcccca tggcggcagc ccagggctct 1320
cccacgcacc ccgcttaccc cagtggacac gccatcaaca acggcgccta catcaccgcg 1380
ctcaaggcgt tcctcgggta cgaggccggc cagaagtgct tccccaaccc cgtggtctcg 1440
aacgacgagg gaaccaagag gatcaaatac aagccctccg gaagggagat tgtgggcgag 1500
tgcgtcaacg agaagggcaa gctcgtcgaa ggcctcacgt acgagggaga gctcaacaag 1560
atcagcgcca acgtgctctt gggaaggtcc cacatcggtg ttcactggag gatggacggc 1620
gtgtacggag cgctcatggg agagaccagc tgcgtccgcc gcctgcagca ggaacttcct 1680
ggcctcccgg aggcccgcga ggttgagggc aagaagcgcc gtggtgacat ccccccggcg 1740
acttacaaat tccgccttta cagcggtaag attcttgagc tctacggcag gaacttgtac 1800
aagctggacg gcaagctctg cgagggggcc ttcaccggcg acgacttctg tgacccgatc 1860
gacgaggacg actacagctc gttcgacgac atcgtcgagg aacacgccca gttctcgctc 1920
cacggacaca ccgagctc 1938

<210> 8

<211> 2046
<212> DNA
<213> Laminaria digitata

<220>
<223> ORF coding for a bromoperoxidase vBPO2

<400> 8
atgaagcgcg ctaggcccac ccagcccggc gcgctgtact ctgtcctctc tctccgacac 60
gtggcgtgtg ccctgtgcgt tgtcgcatgc gctttccttt tgttcgaata tcagatttct 120
tccttgggct tgaccgcttt gctggcggcg ttcgctccct gcctcggcta cgaggagcct 180
cccgagccca cgcagcctct gctcagcggg aacgtgtgca gggtgcgaga ctccctcgac 240
ttccttgacc cggtgccgcg cgcgaaggtg accctgctca agcgcctggc catcgccaag 300
gacgagatat cggttggacc cacttgccac gtcaacaacg gcgacgagga aaacgtcccg 360
ctcttcgctg ggcagtacca caagacgcta ccgcacgaca agttcggtca ggtggacgaa 420
gacgcataca agaagctgct ggagtgcgtc ttcacgagcg acatcaacga gtgcgagaag 480
gtgccctccg gcgccggccg aagaggtggc gcgaagctga ccaacccccct cgggggcacc 540
gcgcaccagg tcaccggtgc tgacagcgac aatgtgttca tcaccacgcc cgacagtctc 600
ctctccgaaa ggctggccgc gcagcaggca gaagtttact ggatggccct gcttagggac 660
atccccttcg gggagttcgc caagaacgac tacgtcaggc tcgccgcaga gaacctgcaa 720
agcctgccgg cgttcaaggg actcaacatt ccccggagcg agggaggaaa gatcgaccct 780
gtcactgatc tgttccgaac gacctggccg ggtgtgacca ccggtcccgt cgtgtctcag 840
ttcatgctgt cggacttcct gatcgactct atcaaggtca ccccgaaggc tgaccccctt 900
accccagggg tggactacat gaccgccttc cagccgtggc tcgacgtaca gaacggggct 960
tccaagcttg aaactacttt cgacgaggag aacccccgct tcatccgcaa cggccgagac 1020
ctggccacta tcgccttgag ggatcaactc tacacggagg ccttccgcgc cgcgctcatc 1080
ctgttcacgg aaggtgcact tggcggcgag gtcgggcctt acgcggaggc cgagcgccag 1140
caggggttcg ccactttcgg ggagccgcac atcctcaccg ccatggcctc ggccagctct 1200
tccacgcgcc acgcgtggta cgctaagtgg caagtccacc gcatgctgcg acccgaggcg 1260
tacggtgccc tggtacataa cacgctcatg agggacgtca tcaccccgct gccggactct 1320
atcctgagga acacggagct cctcaaccga gtggaggtgc acaaccagcg tatgaaccca 1380
gacggcgaga agaccttcct gctccccatg gcggcagccc agggctctcc cacgcacccc 1440
gcttacccca gtggacacgc catcaacaac ggcgcctaca tcaccgcgct caaggcgttc 1500
ctcgggtacg aggccggcca gaagtgcttc cccaaccccg tggtctcgaa cgacgaggga 1560
accaagagga tcaaatacaa gccctccgga agggagattg tgggcgagtg cgtcaacgag 1620
aagggcaagc tcgtcgaagg cctcacgtac gagggagagc tcaacaagat cagcgccaac 1680
gtgctcttgg gaaggtccca catcggtgtt cactggagga tggacggcgt gtacggagcg 1740
ctcatgggag agaccagctg cgtccgccgc ctgcagcagg aacttcctgg cctcccggag 1800
gcccgcgagg ttgagggcaa gaagcgccgt ggtgacatcc ccccggcgac ttacaaattc 1860
cgcctttaca gcggtaagat tcttgagctc tacggcagga acttgtacaa gctggacggc 1920
aagctctgcg agggggcctt caccggcgac gacttctgtg acccgatcga cgaggacgac 1980
tacagctcgt tcgacgacat cgtcgaggaa cacgcccagt tttcgctcca cggacacacc 2040
gagctc 2046

<210> 9
<211> 1872
<212> DNA
<213> Laminaria digitata

<220>
<223> ORF coding for a iodoperoxidase vIPO

<400> 9
atgaaggggc ttgcaggacc agccggtgct atggccgttg tcgcgctcgg gcttgtcccc 60
ggcggagcaa tcgggaaatc tttgcgacaa gagccctctg aaccccgcct aagtggcggc 120
gtggatacgg ctgcatcgcc atcgaaggac accctgaaag ggagtctttc gcgtaagctc 180
caagtcgtca acgatgatgc cctcgatgtt agtggcacgc ccgcagaaag agctgccaac 240
gcgctgaacc agcggatcga atttgcggag acggagttca cggcatccga aggcacgctc 300
caccttaaca acggagaccg ctcatccgcc gccacgttcc acaagtcgct gccgcacgac 360
agcctaggac aggtgaacag cgaggacttt gacctcctca tggagtgcat cgctcaaggt 420

```
gatttcgaca catgcgagct ggtgccggcc ggagacgacg gcaggctgtc caaccccctc    480
gggggtatcg ccgtcgagat ggcgggagcc gccggccccg ctttgaccct ccctccggcc    540
tcagcgatca actccgagga cttggctgct caaatggcgg aacagtactg gatggccttg    600
accagggacg tacctttctc tcagtacggc gaggatgagg cgacagtggc tgcagcagac    660
aacttggcca ccatgcctgg ttttgccgac attgtcgggg tggccgtcga tccggaaacc    720
agaagagcgg atccgcagtc gcagcttttc cggtcctctg ccttcggcgt cgagacaggg    780
cccttcattt cccagctact ggtgaaggac ttcacgattg attctatcac tgtgacgcct    840
atgcagaaga cgtttgcgcc cggagcagac tacatgaccg actacgacga atggctctcc    900
atacagaacg gtggcagccc tgactcggaa gcggacctag acgacgagga ccggtacatc    960
cgcaactccc gcgacctctc taggctggtg gctaccgaca ccgtcaacac ggaggcgtac   1020
cgagccgctc tgattcttct cgacccggac cagggagccg atggccgggc agccatcagc   1080
gctcccggct tgaacggtcc ctacgcggac agcagccgcc aggccggctt cgtcaactac   1140
ggcgtgtctc accttatgag gctcgtcgga accgccgagc tggcccagaa gtccgcgtgg   1200
taccaaaagt ggaacgtgca catgttcgta cgtccggagg ctttcggcgg aagcatccac   1260
aacgtcctct tgggcaaact cgacgtagag atcgccccct cgcttctcaa aaacacggat   1320
ttgctagaca gggtggctgc acggaacggg gaaatcaacg ggcggccagg agtactcgac   1380
cgcacctacc tcctctccca ggccctcccc gaggggtcgc caactcaccc atcgtacccc   1440
gctgggcacg ccacccagaa cggtgcattc gccacggtgc tcaaggccct ggtcgggctg   1500
gagcgtggct ctgtctgctt caatgacccc gtgttccccg acgacgaagg gctgaccctt   1560
ctgccctaca ccggagacga cggaaacaac tgcctaacat tcgagggaga aatcaacaag   1620
ctggccgtca acgtggcatt gggcaggaac atgttgggtg ttcactggag gatcgacagc   1680
gagttgggtt tgctcctcgg cgagacggca gctgtgagga tcctgcagca ggaggccgtg   1740
gcatacccag agaacgcggg atacgagttc cgtctgatgt caggcaagac catcaggctc   1800
gaaaccgacg ggacattctt catcgacgac acgctgtgca gcggggacgc gttcatggga   1860
gctgacctgt gc                                                       1872
```

<210> 10
<211> 20
<212> DNA
<213> Laminaria digitata

<220>
<223> PCR primer

<400> 10
ctgcaggttc tctgcggcga    20

<210> 11
<211> 54
<212> DNA
<213> Laminaria digitata

<220>
<223> PCR primer

<400> 11
ggggacaagt ttgtacaaaa agcaggcttc tacgaggagc ctccctcccg agcc    54

<210> 12
<211> 54
<212> DNA
<213> Laminaria digitata

<220>
<223> PCR primer

<400> 12
ggggaccact ttgtacaaga aagctgggtc ctagagctcg gtgtgtccgt ggag    54

<210> 13
<211> 5
<212> PRT
<213> Laminaria digitata

<220>
<223> Iodoperoxidase partial sequence

<400> 13
Ala Val Asn Val Ala
1               5

<210> 14
<211> 7
<212> PRT
<213> Laminaria digitata

<220>
<223> Iodoperoxidase partial sequence

<400> 14
Ser Ala Pro Gly Ile Asn Gly
1               5

<210> 15
<211> 13
<212> PRT
<213> Laminaria digitata

<220>
<223> Iodoperoxidase partial sequence

<400> 15
Ile Val Ala Ala Asp Thr Val Asn Thr Glu Ala Tyr Arg
1               5                   10

<210> 16
<211> 7
<212> PRT
<213> Laminaria digitata

<220>
<223> Iodoperoxidase partial sequence

<400> 16
Ser Ala Pro Gly Leu Asn Gly
1               5

<210> 17
<211> 13
<212> PRT
<213> Laminaria digitata

<220>
<223> Iodoperoxidase partial sequence

```
<400> 17
Leu Val Ala Ala Asp Thr Val Asn Thr Glu Ala Tyr Arg
1               5                   10
```

## Description

The present invention relates to the technical field of halide oxidation during chemical or biochemical processes, using haloperoxidases from brown algae.

More specifically, the present invention relates to bromide and/or iodide oxidation, using specific and efficient bromo- and iodoperoxidases from *Laminaria digitata.*

Accordingly, the invention concerns bromo- and iodoperoxidases from *Laminaria digitata,* isolated nucleic acids encoding same, as well as methods for preparing these enzymes.

Moreover, the present invention is also directed to the use of such haloperoxidases in a wide range of industrial, pharmaceutical and ecological applications.

Brown algae from the order Laminariales (kelps) accumulate iodine to more than 30,000 times the concentration of this element in seawater, up to levels as high as 1% of dry weight (Küpper *et al.,* 1998). In this respect, Laminariales represent the living organisms that concentrate the most iodine, compared to other living organisms including other orders of brown algae.

Not much is known, however, on the iodine-concentrating mechanisms and on the biological functions of iodine in these kelps and other marine plants. Only one aspect of halogen metabolism, the production of volatile halocarbons (VHCs), has attracted attention, because these compounds, and in particular the iodinated forms, have a significant impact on the chemistry of atmosphere (O'Dowd *et al*., 2002). In the biology of marine algae, VHCs are viewed as defense metabolites, i.e., products of the scavenging of activated oxygen species and/or potent biocides (Borchardt *et al.,* 2001; Potin *et al*., 2002).

Halogen uptake (Küpper *et al.,* 1998), as well as production of haloorganic compounds (Butler, 1998) by marine algae, are thought to involve vanadium-dependent haloperoxidases (vHPOs), also called haloperoxidases (HPOs).

HPOs catalyze the oxidation of halides according to the following reaction:

$$H_2O_2 + X^- + H^+ \rightarrow H_2O + HOX$$

wherein:

$X^-$ represents a halide ion and is $Cl^-$ or $Br^-$ or $I^-$.
HPOs are named according to the most electronegative halide that they can oxidize:

- chloroperoxidases (CPOs) can catalyze the oxidation of chloride as well as of bromide and iodide;
- bromoperoxidases (BPOs) react with bromide and iodide; whereas
- iodoperoxidases (IPOs) are the most specific as they only react with iodide.

The ability of vHPOs to halogenate a broad range of organic compounds of both commercial and pharmaceutical interest, as well as their high stability towards high temperatures, oxidative conditions and in the presence of organic solvents, make them good candidates for use in industrial biotransformations (Vilter, 1995; Butler *et al.,* 2001).

These properties have elicited detailed structural and mechanistic studies on several vHPOs, namely the CPO from the fungus *Curvularia inaequalis* (Messerschmidt and Wever, 1996) and the BPOs from the red algae *Corallina pilulifera* (Shimonishi *et al.,* 1998; Ohshiro *et al.,* 2002) and *Corallina officinalis* (Isupov *et al.,* 2000; Carter *et al.,* 2002) or from the fucalean brown algae *Fucus distichus* (Vreeland *et al.,* 1998) and *Ascophyllum nodosum* (Weyand *et al.,* 1999).

International Patent Application WO 01/53494, in the name of The Regents of the University of California, provides new haloperoxidases having the properties of vanadium BPO (vBPO) from red algal *Corallina* species, said vBPO being obtainable for commercial purposes.

United States Patent US 6,232,457, in the names of Vreeland *et al*., discloses nucleic acids encoding polypeptides corresponding to vBPO from *Fucus distichus* brown algae.

Most of known BPOs from algae exhibit enzymatic activities much lower than those of brown algae, especially Laminariales (Wever *et al*., 1991; Almeida *et al*., 2001).

Nevertheless, until the present invention, no haloperoxidase from Laminariales has yet been provided, at least probably in part because of high difficulties in extracting and purifying enzymes from these organisms.

Moreover, no haloperoxidase specific for iodide has ever been described.

In this context, the present invention provides BPOs and IPO from *Laminaria digitata*, said enzymes having highly specific and highly efficient activities, compared to yet available vHPOs.

A first aspect of the present invention is related to a purified HPO from *L. digitata.*

The term "purified" as used herein means that at least one order of magnitude of purification is achieved, preferably two or three orders of magnitude, most preferably four or five orders of magnitude of purification of the starting material or of the natural material. Thus, the term "purified" as utilized herein does not necessarily mean that the material is 100% purified and that said material thus excludes any other material.
More precisely, by "purified HPO", it is referred to a proteinaceous composition comprising a HPO extracted from *L. digitata* cells or from recombinant host cells, wherein said HPO is purified to any degree relative to its naturally-obtainable state, i.e., wherein said HPO is free from its natural environment. This does not mean in all cases that a "purified HPO" is free from any other compounds or impurities, provided the HPO activity is the only one to be retained by the composition, as may be assessed, for example, by the protein assays described in paragraph 1.4 herein below, or as would be known to one of ordinary skill in the art for the desired HPO.
Substantially pure compositions of at least about 90 to 95% homogeneity are preferred, and 98 to 99% or more homogeneity are most preferred.
Once purified, partially or to homogeneity, the HPOs may then be used as described below.
Extraction and purification of enzymes, including HPOs, from *L. digitata* are known to be difficult and cumbersome by those skilled in the art. Indeed, extraction and purification of HPOs may not be satisfyingly achieved according to standard procedures, but are now made possible using the methods of the present invention (see paragraphs I.2 and II.1 below).
The terms "peptide", "protein" and "polypeptide" refer to a sequence of amino acids or residues having no specific length. Thus, peptides, oligopeptides, polypeptides and proteins are encompassed by this definition. Also covered by this definition, are polypeptides having undergone post-translational modifications such as polypeptides having covalent attachment of glycosyl groups, acetyl groups, phosphate groups, lipid groups, and the like.
In other words, in the context of the invention, the terms "peptide", "protein" and "polypeptide" are used interchangeably to refer to an "amino acid sequence". Such a sequence is preferably that of an enzyme and, more preferably, that of a HPO, especially that of a BPO or a IPO.
The terms "activity", "function", "biological activity", and "biological function" are equivalent and have to be understood as it is well known in the art. Preferably, such an activity is enzymatic. More preferably, this activity is a HPO activity, especially a BPO or IPO activity.
In the context of the invention, HPOs are preferably BPOs and/or IPOs. The appropriate meaning will be clearly and unambiguously deduced by the skilled artisan from the context wherein HPOs are referred to.
According to a first embodiment, the purified HPO as defined above is a BPO from *L. digitata.*
More precisely, a BPO according to the present invention has a sequence selected from:

- SEQ ID No. 1, SEQ ID No. 2; and
- functional fragments and functional variants thereof.

By "functional fragments" of an amino acid sequence of reference having a biological activity of interest (as defined above), it is meant parts of this amino acid sequence of reference, said parts comprising at least all the regions essential for exhibiting the biological activity of the amino acid sequence of reference. These parts of sequences can be of various lengths, provided the biological activity of the amino acid sequence of reference is retained by said parts.
The definition above can be extended to "functional fragments" of a nucleic acid of reference, said nucleic acid of reference encoding a protein having a biological function of interest.
By "functional variants" of an amino acid sequence of reference having a biological activity of interest (as defined above), it is meant proteins that structurally differ from the amino acid sequence of reference but that generally maintain all the essential functional characteristics of said amino acid sequence of reference. A variant of a protein may be a naturally-occurring variant or it may be a variant that is known naturally not to occur. Such non-naturally occurring variants of the reference protein can be made by, for example, mutagenesis techniques on the encoding nucleic acids.
Structural differences may be limited so that the amino acid sequence of reference and the variant may be closely similar overall and, in many regions, identical.
Structural differences may result from conservative or non-conservative amino acid substitutions, deletions and/or additions between the amino acid sequence of reference and the variant. The only proviso is that, even if some amino acids are substituted, deleted and/or added, the biological function of the amino acid sequence of reference is retained by the variant or a biological function of haloperoxidase of interest but different than that of the amino acid sequence of reference is exhibited by said variant. It is known that amino acids are classified in different classes according to the nature of their side groups, for instance, the basic residues lysine, arginine, histidine; the dicarboxylic residues aspartic acid and glutamic acid; etc... Therefore, some amino acids can be interchanged for one another when belonging to the same class, while the properties resulting from the side groups are maintained.
A BPO according to the present invention is encoded by a nucleic acid comprising at least one nucleotide sequence selected from:

- SEQ ID No. 4; SEQ ID No. 5; SEQ ID No. 7; SEQ ID No. 8;
- their complementary sequences; and
- functional fragments and functional variants of SEQ ID No. 4, of SEQ ID No. 5, SEQ ID No. 7, SEQ ID No. 8, and of their complementary sequences.

As used herein, the terms « polynucleotides », « nucleic acids », « nucleotide sequences », and « oligonucleotides » are used interchangeably and include, but are not limited to RNA, DNA, RNA/DNA sequences of more than one nucleotide in either single chain or duplex form.

The nucleotide sequences of the present invention may be prepared from any known method including, but not limited to, any synthetic method, any recombinant method, any ex *vivo* generation method and the like, as well as combinations thereof.

As used herein, the term "complementary" means that, for example, each base of a first nucleotide sequence is paired with the complementary base of a second nucleotide sequence whose orientation is reversed. The complementary bases are A and T (or A and U) or C and G.

"Variants" of polynucleotides according to the present invention include, but are not limited to, nucleotide sequences which are at least 95% identical after alignment to the reference polynucleotide encoding the reference polypeptide. These variants can also have 96%, 97%, 98%, 99%, and 99,999% sequence identity to the reference polynucleotide.

Nucleotide changes present in a variant polynucleotide may be silent, which means that these changes do not alter the amino acid sequence encoded by the reference polynucleotide.

Also encompassed by the term "variants" of a nucleic acid of reference, are polynucleotides which can hybridize to said nucleic acid of reference. Hybridizing polynucleotides can be useful as probes or primers, for example.

For instance, such hybridizing polynucleotides may be at least 10 nucleotides in length or preferably, at least 17 nucleotides in length. They may also be at least 25 or at least 50 nucleotides in length.

In the context of the present invention, hybridizing polynucleotides will preferably hybridize to the nucleic acid of reference under stringent hybridization conditions. One example of stringent hybridization conditions is where attempted hybridization is carried out at a temperature of from about 35°C to about 65°C using a salt solution which is about 0.9 molar. However, the skilled person will be able to vary such conditions appropriate in order to take into account variables such as probe length, base composition, type of ions present, etc...

Identity between nucleotide or amino acid sequences can be determined by comparing a position in each of the sequences which may be aligned for the purposes of comparison. When a position in the compared sequences is occupied by the same base or amino acid, then the sequences are identical at that position. A degree of sequence identity between nucleic acids is a function of the number of identical nucleotides at positions shared by these sequences. A degree of identity between amino acid sequences is a function of the number of identical amino acid sequences that are shared between these sequences.

Since two polynucleotides may each: (i) comprise a sequence (i.e., a portion of a complete polynucleotide sequence) that is similar; and (ii) may further comprise a sequence that is divergent, sequence identity comparisons between two or more polynucleotides over a "comparison window" refers to the conceptual segment of at least 20 contiguous nucleotide positions wherein a polynucleotide sequence may be compared to a reference nucleotide sequence of at least 20 contiguous nucleotides and wherein the portion of the polynucleotide sequence in the comparison window may comprise additions or deletions (i.e. , gaps) of 20 percent or less compared to the reference sequence (which does not comprise additions or deletions) for optimal alignment of the two sequences.

To determine the percent identity of two amino acid sequences or two nucleic acid sequences, the sequences are aligned for optimal comparison. For example, gaps can be introduced in the sequence of a first amino acid sequence or a first nucleic acid sequence for optimal alignment with the second amino acid sequence or second nucleic acid sequence. The amino acid residues or nucleotides at corresponding amino acid positions or nucleotide positions are then compared. When a position in the first sequence is occupied by the same amino acid residue or nucleotide as the corresponding position in the second sequence, the molecules are identical at that position.

The percent identity between the two sequences is a function of the number of identical positions shared by the sequences. Hence % identity = number of identical positions / total number of overlapping positions X 100.

In this comparison, the sequences can be the same length or may be different in length.

Optimal alignment of sequences for determining a comparison window may be conducted by the local homology algorithm of Smith and Waterman (1981), by the homology alignment algorithm of Needleman and Wunsch (1972), by the search for similarity via the method of Pearson and Lipman (1988), by computerized implementations of these algorithms (GAP, BESTFIT, FASTA and TFASTA in the Wisconsin Genetics Software Package Release 7.0, Genetic Computer Group, 575, Science Drive, Madison, Wisconsin) or by inspection.

The best alignment (i.e., resulting in the highest percentage of identity over the comparison window) generated by the various methods is selected.
In other words, the "percentage of sequence identity" is calculated by comparing two optimally aligned sequences over the window of comparison, determining the number of positions at which the identical nucleic acid base (e.g., A, T, C, G, U, or I) occurs in both sequences to yield the number of matched positions, dividing the number of matched positions by the total number of positions in the window of comparison (e.g., the window size) and multiplying the result by 100 to yield the percentage of sequence identity. The same process can be applied to polypeptide sequences.
The percentage of sequence identity of a nucleic acid sequence or an amino acid sequence can also be calculated using BLAST software (Version 2.06 of September 1998) with the default or user defined parameter.
According to a second embodiment, the purified HPO as defined above is a IPO from *L. digitata.*
Such a IPO has a sequence selected from:

- SEQ ID No. 3; and
- functional fragments and functional variants thereof.

The IPO according to the invention is encoded by a nucleic acid comprising at least a nucleotide sequence selected from:

- SEQ ID No. 6, SEQ ID No. 9;
- their complementary sequences; and
- functional fragments and functional variants of SEQ ID No. 6, SEQ ID No. 9, and of their complementary sequences.

The nucleic acids disclosed herein are advantageously contained in a recombinant vector, preferably a recombinant expression vector.
The terms "vector" and "plasmid" relate to the same tool which is useful for performing procedures of molecular biology and genetic recombination. Such a tool is commonly used and very well known in the art.
A "recombinant expression vector" is a recombinant vector that enables a nucleic acid contained therein to be expressed.
This recombinant vector, especially this recombinant expression vector, is advantageously contained in a recombinant host cell.
A "recombinant host cell" according to the invention is a cell chosen from:

- prokaryotic organisms, such as eubacteria, and preferably *Escherichia coli;* and
- eukaryotic organisms, such as yeasts, insects, heterokonts, oomycetes and brown algae.

In a second aspect, the present invention concerns an isolated nucleic acid encoding a BPO from *L. digitata,* said isolated nucleic acid comprising a sequence selected from:

- SEQ ID No. 4; SEQ ID No. 5; SEQ ID No. 7; SEQ ID No. 8;
- their complementary sequences; and
- functional fragments and functional variants of SEQ ID No. 4, of SEQ ID No. 5, SEQ ID No. 7, SEQ ID No. 8, and of their complementary sequences.

The term "isolated" as used herein means that a nucleic acid has been removed from its original environment in which it is naturally present. Indeed, a polynucleotide, when present in a plant, mammal or animal in its naturally state, is not considered to be isolated, whereas the same polynucleotide, when separated from the adjacent nucleic acid sequences in which it is naturally inserted in the genome of said plant, mammal or animal, is considered as being "isolated".
The term" 'isolated" is not meant to exclude artificial or synthetic mixtures with other compounds, or the presence of impurities which do not interfere with the biological activity and which may be present, for example, due to incomplete purification, addition of stabilizers or mixtures with pharmaceutically acceptable excipients and the like.
In a third aspect, the present invention is directed to an isolated nucleic acid encoding a IPO from *L. digitata,* said isolated nucleic acid comprising a sequence selected from:

- SEQ ID No. 6, SEQ ID No. 9;
- their complementary sequences; and
- functional fragments and functional variants of SEQ ID No. 6, SEQ ID No. 9, and of their complementary sequences.

Recombinant vectors and recombinant host cells as defined above, that contain the aforementioned nucleic acids, are also encompassed by the present invention.

In a fourth aspect, the invention relates to a BPO from *L. digitata* encoded by an isolated nucleic acid comprising a sequence selected from:

- SEQ ID No. 4; SEQ ID No. 5; SEQ ID No. 7; SEQ ID No. 8;
- their complementary sequences; and
- functional fragments and functional variants of SEQ ID No. 4, of SEQ ID No. 5, SEQ ID No. 7, SEQ ID No. 8, and of their complementary sequences.

In particular, said BPO has a sequence selected from:

- SEQ ID No. 1, SEQ ID No.2; and
- functional fragments and functional variants thereof.

In a fifth aspect, the present invention concerns a IPO from *L. digitata* encoded by an isolated nucleic acid comprising a sequence selected from:

- SEQ ID No. 6, SEQ ID No. 9;
- their complementary sequences; and
- functional fragments and functional variants of SEQ ID No. 6, SEQ ID No. 9, and of their complementary sequences.

Especially, a IPO of the invention has a sequence selected from:

- SEQ ID No. 3; and
- functional fragments and functional variants thereof.

According to a sixth aspect, the invention is related to a method for preparing HPO, especially BPO and IPO, from *L. digitata.*

Such a method comprises at least:

a) cloning at least one nucleic acid as defined above, into a recombinant expression vector;
b) transforming a recombinant host cell with said recombinant expression vector ; and
c) expressing said at least one nucleic acid from said recombinant host cell.

The HPO thus obtained at the end of step c) can be advantageously purified.

The recombinant host cell used in steps b) and c) is as defined above.

In a seventh aspect, the present invention concerns uses of HPOs from *L. digitata*.

These enzymes can be used in a number of industrial, pharmaceutical, and ecological applications.

Indeed, the HPOs of the invention can suitably be used for any purpose to which prior art HPOs are used, wherein bromide and/or iodide are used.

In a first embodiment, the HPOs according to the present invention are useful for halogenating organic compounds, such as pesticides, drugs, adhesives, and the like.

In a second embodiment, the above defined HPOs can be used for trapping halogens, for instance during water treatment.

In a third embodiment, the HPOs form *L. digitata* can be used for *in situ* producing antibiotic compounds, for example in anti-fouling treatments of boats.

The present invention is illustrated, while not being limited, by the following figures:

**FIG.1.** In gel haloperoxidase assay of protein extracts from *L. digitata* sporophytes under non-denaturing conditions. Polyacrylamide gels were loaded with haloperoxidase fractions (1.6 μg of proteins) and stained for iodoperoxidase activity (lane 1), bromoperoxidase activity (lane 2) and chloroperoxidase activity (lane 3). Bromoperoxidase activity bands are referred to as BPOa-f and the iodoperoxidase band is indicated as IPO.

**FIG.2.** Purification of iodoperoxidase and bromoperoxidase from *L. digitata* followed by activity staining. (A) Polyacrylamide gel electrophoresis of the iodoperoxidase fraction under non-denaturing conditions. Lane 1. Aqueous salt/polymer two-phase system extract (130 ng) stained for iodoperoxidase activity. Lanes 2-3. IPO fraction purified by semi-preparative electrophoresis (130 ng), stained for iodoperoxidase activity and with silver nitrate, respectively. (B) Polyacrylamide gel electrophoresis under non-denaturing conditions of the bromoperoxidase fractions.

Lane 1. Phenyl-sepharose partially purified extract, from which five distinct bromoperoxidase activity bands, annotated in the left of the gel, were extracted by electroelution from another gel run under identical conditions. Lane 2-6. PAGE patterns of the proteins electroeluted from the BPOa, BPOb, BPOc, BPOd and BPOe-f bands, respectively. The gel was loaded with 200 ng of proteins and stained for bromoperoxidase activity. The relative molecular weights indicated on the right were established, using standard proteins, by non-denaturating gel electrophoresis analyses under a range of acrylamide concentrations as described by Hedrick *et al.* (1968), and confirmed by fractionation of haloperoxidase activities by gel filtration chromatography (C). SDS-PAGE analyses of the purified IPO (lanes 1-2) and BPO (lanes 3-4) from *L. digitata.* Proteins were denatured in the absence (lanes 1 and 3) or in the presence of β-mercaptoethanol (lanes 2 and 4). Each lane was loaded with 30 ng of IPO and 100 ng of BPO and stained with silver nitrate. The relative molecular weights indicated on the right were established using a standard protein kit.

**FIG.3.** Iodoperoxidase specific activity of the purified IPO and BPO as a function of pH at 20 °C (A) and as a function of temperature at pH 6.2 (B). Error bars indicate standard deviations (n=3).

**FIG.4.** MS/MS spectrum and deduced amino acids of the 1371.86 Da tryptic peptide from purified BPO ($NH_2$-I/L-A-A-E-N-I/L-K/Q-G-I/L-P-A-F-K/Q-COOH). Mass data are the average of seven MS/MS analyses. A star on the right of the spectrum indicates the precursor ion.

**FIG.5.** Multiple amino acid sequence alignment of known vanadium bromoperoxidases from brown algae. Abbreviations : An-vBPO, *Ascophyllum nodosum* vBPO, Swiss-Prot # P81701; Fd-vBPO, *Fucus distichus* vBPO, NCB Accession # AAC35279; Ld-vBPO1 and Ld-vBPO2, *Laminaria digitata* vBPO1 and vBPO2, EMBL Accession # AJ491786 and EMBL Accession # AJ491787, respectively. The conserved residues in all of the four sequences are shown in white capitals on a black background, and the residues that occur in at least three of the sequences are in black capitals on a grey background. The amino acid residues associated with the active site vanadium cofactor are indicated by the symbol # and the cysteine residues involved in disulphide bridges according to the An-vBPO structure (Weyand *et al.* 1999) are marked with * above the alignment. The putative cleavage site of signal peptides in Ld-vBPO is referred to by a triangle. The seven peptides identified by LC-MS from the purified bromoperoxidases of *L. digitata* are underlined, with the four peptides sequenced by LC-MS/MS out in full below the alignment. The peptide used to produce an antipeptide to the vBPO from *L. digitata* is boxed. Arrows point out the intragenic probe used for Southern blot.

**FIG.6.** Southern blot hybridization of *L. digitata* genomic DNA with an intragenic probe from *L. digitata* vBPO cDNA. Lanes 1-3 contain *L. digitata* genomic DNA digested with *Sal*I, *Sal*I/*Xho*I*,* and *Xho*I, respectively. The intragenic probe is shown in Fig. 5.

**FIG.7.** Western blot hybridization of the purified bromo- (lane 1) and iodoperoxidase (lane 2). Each lane contains 200 ng of protein, treated with SDS and β-mercaptoethanol. Blots were stained with antibodies directed against *L. digitata* vBPO (A) or against *A. nodosum* vBPO (B).

**FIG.8.** Structure model of the vBPO1 monomer from *L. digitata* (A) compared to the ribbon-type representation of the vBPO monomer from A. *nodosum* (B). Secondary structure assignments : α-helices are shown in grey, and β-strands and coils in black. The N- and C-terminal ends of *L. digitata* vBPO1 protein [amino acid (aa) residues 1-42 and 583-646, respectively] were not modelized due to the lack of homology with the vBPO protein of *A. nodosum.* The additional α-helix in *L. digitata* vBPO1 is marked by an arrow.

**FIG.9.** Expression of *L. digitata* vBPO1 in *E. coli.* (A) Denaturing SDS-PAGE analysis (8% polyacrylamide) of *E. coli* protein extracts (25 μg), stained with Coomassie Blue, from inclusion bodies fractions (lanes 1-4) and from cytoplasmic fractions (lanes 5-8), under the following culture conditions : lanes 1 and 5, uninduced culture; lanes 2 and 6, culture induced with 150 mM NaCl; lanes 3 and 7, uninduced culture supplemented with 3% EtOH; lanes 4 and 8, culture induced with 150 mM NaCl and supplemented with 3% EtOH. (B) In-gel iodoperoxidase assay of the purified BPO extract (100 ng) from *L. digitata* (lane 1) and of the recombinant protein extracts (50 μg) from the cytoplasmic fractions, under the following culture conditions : lane 2, uninduced culture; lane 3, culture induced with 150 mM NaCl; lane 4, uninduced culture supplemented with 3% EtOH; lane 5, culture induced with 150 mM NaCl and supplemented with 3% EtOH. Note that, in the original gels, the bromoperoxidase activity did appear as one double-band. Arrows indicate the bromoperoxidase bands.

The present invention will be better understood in the light of the following detailed description of experiments, including examples. Nevertheless, the skilled artisan will appreciate that this detailed description is not limitative and that various modifications, substitutions, omissions, and changes may be made without departing from the scope of the invention.

## I. EXPERIMENTAL PROCEDURES

*I.1 Plant Material*

*L. digitata* young sporophytes (blade length less than 25 cm) were collected from the shore in the vicinity of Roscoff (Brittany, France), and maintained under a 12h:12h Light-Dark cycle in running seawater at 17 °C, or immediately frozen in liquid nitrogen and kept at -80 °C. Gametophyte cultures were maintained under red light at 15 °C in Provasoli's enriched filtered seawater.

*I.2 Purification of vanadium-dependent haloperoxidases*

As the algae from the Laminariaceae family are extremely rich in alginates and polyphenolic compounds, an aqueous salt/polymer two-phase system developed by Vilter (1994) was used for the extraction of haloperoxidases. Briefly, 120 g of *L. digitata* sporophytes were powdered in liquid nitrogen and extracted using 20% (w/v) $K_2CO_3$ (100 ml per aliquots of 20 g FW) at room temperature and gently shaken for 3 h. Then, polyethylene glycol 1550 Da (13% w/v) was added and the preparation was shaken overnight at 4 °C. The two phases were separated by centrifugation at 5,000 x *g* for 15 min, and 6% (w/v) $(NH_4)_2SO_4$ and 3 volumes of acetone were added to the top phase. After 1 h at -20 °C, the protein extract was pelleted by centrifugation at 10,000 x *g* for 30 min, dissolved in 50 mM Tris-HCl, pH 9.0 buffer, and dialyzed overnight against the same buffer. The extract was then adjusted at 30% saturation with $(NH_4)_2SO_4$ and loaded on a Phenyl-sepharose CL4B hydrophobic interaction column, equilibrated with 30% $(NH_4)_2SO_4$, 50 mM Tris-HCl, pH 9.0. Proteins were eluted by a decreasing linear gradient down to salt-free Tris buffer. The active fractions (see paragraph 1.4 below for haloperoxidase activity assay) were pooled, dialyzed against 50 mM Tris-HCl, pH 9.0, and concentrated by filtration (YM-10, Millipore, Saint-Quentin, France) on stirred ultracentrifugation cells (model 8200, Amicon, Millipore, Saint-Quentin, France). For the final purification of native enzymes, semi-preparative electrophoresis (model 491 Prep Cell, Bio-Rad, Marnes La Coquette, France) or electro-elution (model 422 Electro-eluter, Bio-Rad, Marnes La Coquette, France) was used according to the manufacturer's recommendations.

Proteins of *L. digitata* gametophytes were extracted in 50 mM Tris-HCl, pH 9.0, 15 mM $MgCl_2$, 2 mM DTT buffer, containing 0.5% polyvinylpolypyrolidone and 0.5% Nonidet P-40. After sonication (3 times, 30 s), the pellet was discarded by centrifugation at 10,000 x g for 10 min and the protein extract was recovered from the supernatant. Protein concentrations were determined using the Bio-Rad (Marnes La Coquette, France) Coomassie Protein Assay (Bradford, 1976) with bovine serum albumin as standard (Sigma-Aldrich, Saint-Quentin Fallavier, France) and the holoenzymes were re-constituted by adding 2 mM $NaVO_3$.

*I.3 Polyacrylamide Gel Electrophoresis*

Gel electrophoresis was carried out using 9% polyacrylamide slab gels according to Laemmli (1970). SDS-samples, with β-mercaptoethanol or not, were denatured by boiling at 100 °C for 10 min. Proteins were electrophoresed together with protein molecular mass standards (Bio-Rad, Marnes La Coquette, France) and stained with Coomassie brilliant blue R-250 or using the Silver Staining Kit Protein (Amersham Biosciences, Saclay, France).

*I.4 Enzyme Activity Assays*

Haloperoxidase activities were detected on non denaturing gels, soaked with 100 mM potassium phosphate buffer (pH 7.4), in the presence of 0.1 mM *o*-dianisidine, 0.45 mM $H_2O_2$, and 10 mM KI, KBr or KCl for revealing iodoperoxidase, bromoperoxidase or chloroperoxidase activities, respectively (Jordan and Vilter, 1990). In order to detect haloperoxidase activity on denaturating gels (see above), SDS was removed by washing the gel 4 times for 10 min in Tris-glycine buffer containing 0.1% Igepal CA-630 (Sigma Aldrich).

Bromoperoxidase activity was measured spectrophotometrically by monitoring at 290 nm the conversion of monochlorodimedone (MCD, 20.0/cm/mM) into mono-chlorobromodimedone (0.2/cm/mM) at pH 6.1 (Hager *et al.,* 1966). Specific activities are expressed in unit per milligram of protein, where one unit of bromoperoxidase activity is defined as the amount required for brominating one micromole of MCD per min. Iodoperoxidase activity was recorded at pH 6.2 following the conversion of iodide into triiodide (26.4/cm/mM) at 350 nm using 100 ng of proteins (Vilter, 1984). The differences between the non-enzymatic and the non-enzymatic-plus-enzyme reactions were calculated for each sample. The specific activity is expressed in unit per milligram of protein, where one unit of iodoperoxidase activity is defined as the amount required for consuming one micromole of $H_2O_2$ per min. For optimal pH determination, iodoperoxidase specific activities were measured in the same conditions at 20 °C, using the following buffers: 0.1 M sodium acetate (pH 4.0-5.5), 0.1 M MES (pH 6.8-7.2) or 0.1 M HEPES (pH 6.8-7.2). For thermostability study, proteins were maintained at the appropriate temperature for 10 min before the iodoperoxidase assay.

*I.5 Molecular weight determination of the native enzymes*

Using non denaturating gel electrophoresis, protein samples were run under a range of acrylamide concentrations from 4% to 14% (Hedrick and Smith, 1968). A molecular size standard curve was established using standard proteins (albumin, aldolase, catalase, ferritin, and urease from Amersham Biosciences, Saclay, France), which were electrophoresed under the same conditions. The relative molecular mass of native proteins was also determined by FPLC/gel filtration chromatography (Superdex 200 HR 10/30 column, Amersham Biosciences, Saclay, France) with a mobile phase consisting of 100 mM NaCl in 50 mM Tris-HCl, pH 9.0. Two hundred µl were injected for each sample and the elution was performed at 500 µl/min. Standard proteins (Amersham Biosciences, Saclay, France) were used for column calibration.

*I.6 Mass spectrometry analyses*

Phenyl-sepharose haloperoxidase fractions were separated by FPLC/gel filtration chromatography (Superdex 200 HR) followed by a 9% SDS-PAGE. The in-gel digestion by trypsin was performed on excised activity bands as described by Rabilloud *et al*. (2001).

Nanoscale capillary liquid chromatography-tandem mass spectrometric (nano-LC-MS/MS) analyses of the digested proteins were performed using a CapLC capillary LC system (Micromass, Manchester, UK) coupled to an hybrid quadrupole orthogonal acceleration time-of-flight tandem mass spectrometer (Q-TOF II, Micromass Manchester, UK). The LC-MS union was made with a liquid junction (Micromass Manchester, UK) fitted on a ZSPRAY (Micromass, Manchester, UK) interface. Chromatographic separations were conducted on a reversed-phase (RP) capillary column (Pepmap C18, 75 µm i.d., 15 cm length, LC Packings) with a 200 nl/min flow. A linear gradient from 95% A ($H_2O$ / 0.05% HCOOH) to 45% B (acetonitrile / 0.05% HCOOH) for 35 min followed by a linear gradient to 95% B for 1 min and finally followed by an isochratic step at 95% B during 4 min. Mass data acquisitions were piloted by MassLynx software (Micromass, Manchester, UK) using automatic switching between MS and MS/MS modes. The MS data were acquired from m/z = 300 to m/z = 1500 and MS/MS data from m/z = 50 to m/z = 2000. MS scans were performed during 1 s and followed by 4 MS/MS scans of 1 s each. Fragmentation of precursor was performed using collision with argon gas. The collision energy was selected automatically for each precursor ion depending on precursor ion mass (between 20 and 100 ev). The m/z scale was calibrated by using the synthetic PolyAlanine ions.

Micro-LC-MS/MS experiments (Sauber *et al.,* 2002) were performed using an ion trap mass spectrometer Esquire 3000+ (Bruker-Daltonik GmbH, Bremen, Germany) coupled to an Agilent 1100 Series capillary LC system (Agilent Technologies, Palo Alto, US). In the column-switching system, the 6-port valve from the 1100 column compartment and an additional 1100 binary pump were used. The complete system was fully controlled by ChemStation5. The samples were loaded onto a 0.3 × 35 mm ZORBAX SB-C18 column at a flow rate of 50 µl/min with $H_2O$ / 0.1% HCOOH for 5 min. The 6-port valve of the column compartment was then switched and the trapped tryptic peptides were backflushed onto the analytical column with a gradient consisting of $H_2O$ / 0.1% HCOOH and acetonitrile / 0.1% HCOOH. The analytical column was a ZORBAX 300 SB-C18 0.3 × 150 mm and the flow rate was set to 4 µl/min. The gradient profile used consisted of a linear gradient from 95% A ($H_2O$ / 0.1% HCOOH) to 60% B (acetonitrile / 0.1% HCOOH) for 60 min followed by a linear gradient to 80% B for 5 min and finally followed by an isochratic step at 80% B for 5 min. The ion trap mass spectrometer was operated with the electrospray (ESI) source, positive ions, and data-dependent auto-MS/MS mode to generate the highest possible amount of MS and MS/MS informations during a single chromatographic run. Trap drive was set at 100 m/z. For auto-MS/MS experiments, the two most important ions of each MS spectrum were fragmented by applying a resonance frequency on the end-cap electrodes (peak-to-peak amplitude from 0.3 to 2.0 V) matching the frequency of the selected ions. Fragmentation of the precursor ions occurred in the Ion Trap due to collisions with Helium buffer-gas (pressure $5.10^{-3}$ mbar). Scanning was performed in the standard resolution mode at a scan rate of 13000 m/z/s on the scan range from 50 to 2000 m/z for MS and from 250 to 2000 for MS/MS mode. A total of 15 scans $MS^2$ were averaged to obtain a mass spectrum. Calibration of the Ion Trap analyser was performed by using multiply-charged ions mixture made with the following peptides : Arg-Leu-Enkephalin, Angiotensin, Substance P, Bombesin, and ACTH, with monoisotopic masses at m/z = 712.378 (1+) and 356.689 (2+), m/z = 1046.542 (1+) and 523.771 (2+), m/z = 1347.736 (1+) and 674.368 (2+), m/z = 1620.807 (1+) and 810.903 (2+), m/z = 1233.099 (2+), respectively.

Mass data collected during the nano- and micro-LC-MS/MS analyses were processed and converted into PKL and MGF (Mascot Generic File) files, respectively, and then submitted to the search software MASCOT (Matrix Science, London, UK), with a tolerance on mass measurement of 0.5 Da. The peptide sequences were obtained by mass spectrometry de *novo* sequencing. Data from the nano-LC-MS/MS analysis (Q-TOF II) were deconvoluted with the algorithm Maxent3 (Micromass, Manchester, UK).

*I.7 cDNA Isolation, Cloning and Sequencing*

The longest of the vanadium-dependent bromoperoxidase (vBPO) EST, LamdiSest169est (NCB Accession # AW400475), previously identified in *L. digitata* sporophytes (Crépineau *et al.,* 2000), was subcloned in *Sac*I pBluescript SK vector (Stratagene, La Jolla, US). Plasmid DNA was purified with the GenElutePlasmid Miniprep kit (Sigma-Aldrich, Saint-Quentin Fallavier, France) and sequenced on both strands using the Vistra Thermosequenase core sequencing kit on a Vistra automated DNA sequencer (Amersham Biosciences, Saclay, France). The *L. digitata* sporophyte cDNA λZAP II library (1) was used as a template in polymerase chain reaction to amplify the 5'end of the BPO cDNA using pBluescript universal primer (forward) and a 5'end specific primer from the LamdiSest169est (reverse; 5'-CTGCAGGTTCTCTGCGGCGA-3' SEQ ID No.10). The 1000-bp-PCR fragment obtained was cloned in the pCR 2.1-TOPO vector (Invitrogen, Cergy Pontoise, France) and labeled with [$\alpha^{32}$P] dCTP using the Megaprime labeling kit (Amersham Biosciences, Saclay, France) to screen the *L. digitata* sporophyte cDNA library. Positive cDNA clones were subcloned by PCR using insert-specific oligonucleotides in the pCR 2.1-TOPO vector (Invitrogen, Cergy Pontoise, France) and were sequenced on both strands.

*I.8 Sequence and Structural Analyses*

Primary sequence analysis and translation were carried out using the DNAMAN program version 4.15. Prediction of protein sorting signal and of signal peptide cleavage site were performed using the TargetP, SignalP and PSORT programs, respectively; *in silico* tryptic digestion of protein sequences was performed using the PeptideCutter program (programs available on the www.expasy.org server and the www.cbs.dtu.dk/services site). The vBPO protein sequences were aligned using DNAMAN and displayed using GENEDOC (Nicholas, K.B, free access on www.psc.edu/biomed/genedoc). The protein structure from *L. digitata* vBPO was predicted using the SWISS-MODEL program based on the 3-D structure of *A. nodosum* vBPO (NCB Accession # 1 QI9A). The 3-D representations were visualized by the Swiss-PdbViewer software (free access on www.expasy.org server).

*I.9 Southern analyses*

Genomic DNA was isolated from frozen *L. digitata* sporophytes as described by Apt *et al.* (1995). Digested DNA (5 μg/lane) was electrophoresed on a 0.8% agarose gel and blotted onto nylon filters. The vBPO intragenic probe was prepared from an 800-bp *Eco*RI-*Sac*I fragment of LamdiSest169est (corresponding to Leu182-Leu412 in vBPO1, see Fig. 5). Southern blot hybridization, probe labeling and detection were performed using the Gene Images random prime labeling module kit according to the manufacturer's recommendations (Amersham Biosciences, Saclay, France).

*I.10 Immunoblotting*

Denaturated proteins were resolved on a 9% SDS-PAGE and transferred onto a nitrocellulose membrane (Bio-Rad, Marnes La Coquette, France). The membrane was blocked in phosphate-buffered saline pH 7.5, containing 0.01 % Tween 20 (PBS-T) and 5% (w/v) milk, incubated with the bromoperoxidase antibodies (see below) overnight at 4 °C, washed 3 times in PBS-T and then incubated for 60 min at room temperature with a goat anti-rabbit IgG coupled to horseradish peroxidase. The detection was performed with ECL Western Blotting System (Amersham Biosciences, Saclay, France). The polyclonal antibodies against the vBPO isoenzyme-I from *A. nodosum* were kindly supplied by H. Vilter (1984) and used at a 1:2,500 dilution. A polyclonal antipeptide to vBPO of *L. digitata* was raised in rabbits against a synthetic peptide (see Fig. 5) by Eurogentec, and used at 1:1,700 dilution.

*I.11 Expression of* L. digitata *vBPO in* E. coli

A 1836-bp fragment from the putative mature protein v-BPO1 was amplified using the primers 5'-GGGGACAAGTTTGTACAAAAAGCAGGCTTCTACGAGGAGCCTCCCTC CCGAGCC-3' (SEQ ID No.11) (forward) and 5'-GGGGACCACTTTGTACAAGAAAGCTGGGTCCTAGAGCTCGGTGTGTC CGTGGAG-3' (SEQ ID No.12) (reverse, *Att*P1 and *Att*P2 recombination sites of the Gateway system are underlined, Invitrogen, Cergy Pontoise, France). The PCR product was inserted into the pDEST17 expression vector and amplified in DH5α*E. coli* strain, for DNA replication and sequence verifications, and in E.coli BL21-SI (Novagen, Darmstadt, Germany), for expression studies. A 2-ml overnight preculture of the transformed bacteria was used to inoculate a 250-ml culture of LBON medium (LB medium without salts) containing 100 μg/ml of ampicilline. Recombinant bacteria were grown at 37 °C until 0.5 $OD_{600}$, then with 150 mM NaCl for 16 h at 30 °C, in the presence or in the absence of 3% ethanol (Thomas and Baneyx, 1997). Cytoplasmic and membrane proteins, hereafter referred to as the cytoplasmic fraction, were extracted from the bacterial pellet by solubilization in B-PER detergent (Pierce, Rockford, US), and the extract was supplemented with 1 mM PMSF (anti-protease protection) and 2 mM $NaVO_3$ (apoenzyme reactivation). Aggregated proteins from inclusion bodies were solubilized by 6 M urea in 50 mM Tris pH 8.0.

## II. RESULTS

*II.1 Purification of haloperoxidases from* L. digitata *sporophytes*

Haloperoxidases from *L. digitata* were extracted using an aqueous salt/polymer two-phase system (Vilter, 1994) and analyzed by non-denaturing electrophoresis followed by an in-gel activity assay (Fig. 1). Haloperoxidase activities were apparent only after adding orthovanadate to the protein extract, indicating that these enzymes are vanadium-dependent. Whereas no chloroperoxidase activity was detected, at least 6 major bands (referred to as BPOa-f) with bromoperoxidase activity were revealed in the presence of either bromide or iodide. A single enzyme activity specific for the oxidation of iodide (referred to as IPO for iodoperoxidase) was apparent (Fig. 1).

Haloperoxidases were further fractionated by hydrophobic interactions on a phenyl-sepharose CL4B column. The IPO activity was then purified to homogeneity by semi-preparative electrophoresis (Fig. 2A). The bands with BPO activity were excised from the non-denaturing electrophoresis gel and electro-eluted separately. When re-analyzed by native gel electrophoresis, however, every band again showed a same, complex pattern (Fig. 2B). Under denaturing conditions (SDS-polyacrylamide gel electrophoresis), IPO ran as a single protein with an apparent molecular mass of 75 kDa, and as a protein of ca 80 kDa in the presence of β-mercaptoethanol (Fig. 2C lanes 1, 2). Upon SDS-PAGE analysis, all of the BPOs were also resolved as one single band, each with relative molecular mass of 145 kDa. In the presence of β-mercaptoethanol they again appeared as a single band, yet with a molecular mass of ca. 70 kDa (Fig. 2C lanes 3, 4).

*II.2 Biochemical properties of haloperoxidases from* L. digitata

The specific activities of *L. digitata* haloperoxidases are summarized in Table I hereunder.

IPO and BPO were purified on a phenyl-sepharole column followed by semi-preparative electrophoresis for IPO and by electro-elution for BPO (see paragraph I.2 above).

TABLE I

| *Sample* | *Specific Activity [U/mg]* | |
|---|---|---|
| | *Bromoperoxidase* | *Iodoperoxidase* |
| Gametophyte crude extract | 0.7 | 0 |
| Sporophyte extract | 2.6 | 10.4 |
| Pure sporophyte BPO | 42 | 62 |
| Pure sporophyte IPO | 0 | 310 |

In contrast to the sporophytes, a weak bromoperoxidase activity and no iodoperoxidase activity was detected in the crude protein extracts from the gametophytes of *L. digitata.* The IPO purified from the sporophytes displayed a specific activity of 310 U/mg towards iodide at pH 6.2. The purified BPO fraction had a lower efficiency towards iodide, in the iodoperoxidase assay. The purified haloperoxidases displayed the same pH optimum, at around 5.5 (Fig. 3). At this pH, iodoperoxidase specific activities were 1200 U/mg for IPO and 180 U/mg for BPOs. Upon heating for 10 min, the purified IPO and BPOs remained fully active up to 60 °C (Fig. 3), with the high molecular-mass BPOs retaining the highest activity up to 80 °C (data not shown).

*II.3 Mass spectrometry analyses of excised BPOs and IPO*

Mass spectrometry analyses were carried out for three of the bromoperoxidase bands, BPOb-d, and for the iodoperoxidase. As the N-terminal sequences of these proteins were blocked, they were digested by trypsin and analyzed by nano- or micro-LC-MS/MS. Very similar MS data were obtained for the three BPOs and among those data, an identical set of eleven peptides was identified in BPOb-d tryptic digests.

The results of LC-MS-MS analyses of purified IPO and BPO tryptic digests are shown and are compared with the theoretical tryptic digest of vBPO1 from *L. digitata* in Table II hereunder. Columns 1 and 2 of Table II refer to the main precursor ions observed by LC-MS for IPO (column 1) and for the BPO bands (column 2). The observed monoisotopic masses of BPO peptides (column 2) were compared with the calculated vBPO1 tryptic digest masses (column 3) and accuracies are reported in column 5 of Table II. The peptides, partially or fully sequenced by LC-MS/MS, are shown in bold characters.

TABLE II

| **IPO** | **BPOb-d** | | **vBPO1** | |
|---|---|---|---|---|
| *MH⁺(obs.) Da* | *MH⁺(obs.) Da* | *MH⁺(calc.) Da* | *Tryptic peptide residues* | ΔM *ppm* |
| 1197.78 | 1856.96 | 1856.84 | 110-124 | 65 |
| 1423.78 | 3326.80 | 3326.65 | 137-168 | 45 |
| 983.58 | 1371.86 | 1371.76 | 198-210 | 73 |
| 1453.82 | 1350.60 | 1350.62 | 287-297 | 15 |
| 875.54 | 1142.80 | 1142.55 | 312-320 | 219 |
| 1646.88 | 1338.82 | 1338.76 | 396-407 | 45 |
| 1524.84 | **1083.80** | 1083.55 | 463-472 | 231 |
| **946.58** | **1563.80** | 1563.71 | 473-486 | 57 |
| 1151.62 | 1464.80 | 1464.76 | 508-520 | 27 |
| | 942.64 | 942.57 | 521-529 | 74 |
| | 1350.80 | 1350.74 | 555-566 | 44 |

As illustrated in Fig. 4, four peptides were fully sequenced, resulting in identical sequences for all of the three BPO bands. None of the eleven peptide masses observed in BPO tryptic digests was found in the IPO tryptic digest, which markedly differed from the BPOs by its mass spectra (Table II above) and by the partial sequences obtained by *de novo* sequencing [AVNVA (SEQ ID No.13), SAPGI/LNG (SEQ ID No.14 and SEQ ID No.16), I/LVAADTVNTEAYR (SEQ ID No.15 and SEQ ID No.17)].

*II.4 Molecular and immunological characterization of L. digitata bromoperoxidases*

In an EST analysis of the life cycle stages of *L. digitata,* one contig from twelve EST reconstructed a partial cDNA showing high homology with the vBPO of *F. distichus* (Crépineau *et al.,* 2000). The cDNA library from *L. digitata* sporophytes was thus screened for full-length vBPO cDNAs using a PCR-amplified probe designed from the 5' end of the partial cDNA, yielding several positive clones, which were mapped by restriction analyses. The two longest inserts, referred to as vBPO1 (3379 bp; EMBL Nucleotide Sequence Database accession number AJ491786) and vBPO2 (3420 bp; EMBL Nucleotide Sequence Database accession number AJ491787), were fully sequenced. They encoded two distinct full-length cDNA, with short 5'UTRs (104 bp and 36 bp, respectively), and with large 3'UTRs (1337 bp and 1338 bp, respectively).

The two cDNAs had 99.2% and 99.5% identity at the nucleotide and protein levels, respectively. They mainly differed in their 5'ends, vBPO1 presenting a putative 20-aa signal peptide with the cleavage site after Gly-20 whereas vBPO2 harbored a longer one (56 aa), with the cleavage site located at Gly-56. The putative mature proteins had molecular masses of 68 953 Da (vBPO1) and 68 957 Da (vBPO2), differing by only 3 aminoacids, Ile-75/Val-111, Gly-205/Ser-241, Val-365/Ser-401 (Fig. 5). The vBPO protein sequences from *L. digitata* presented 38% and 40% identity with those from *F. distichus* and *A. nodosum,* respectively (Fig. 5). In particular, *L. digitata* vBPOs differed from the other brown algal bromoperoxidases by longer C-terminal ends, including 17 aa-long insertions, between Gly-494 and Gly-511 (numbering of vBPO1). They featured, however, the two catalytic histidine residues as well as all of the residues known to be involved in the fixation of vanadium (Weyand *et al*., 1999).

The eleven peptides characterized by LC-MS on the purified bromoperoxidases, corresponded with a good accuracy to those identified by *in silico* tryptic digestion of vBPO protein sequences (Table II above) and they homogeneously covered 22% of the vBPO mature proteins (Fig. 5). Three of the four peptides fully sequenced by LC-MS/MS were present in both vBPO sequences. The fourth peptide (Fig. 4) was only present in vBPO1. In this sequence vBPO2 departed from vBPO1 by replacement of glycine by serine (Fig. 5). In a Southern blot analysis with the vBPO intragenic probe a large number of hybridization bands was detected in *L. digitata* (Fig. 6). A synthetic peptide (see box in Fig. 5) was used to produce a polyclonal antibody against *L. digitata* vBPOs. This antibody cross-reacted with the purified BPOs but not with the purified IPO. This latter enzyme, however, was specifically recognized by antibodies against the vBPO from *A. nodosum* (Fig. 7).

*II.5 3-D structure of* L. digitata *bromoperoxidases*

The 3-D structure of *L. digitata* vBPO1 (Fig. 8) was modelized from the homo-dimeric structure of *A. nodosum* vBPO (Weyand *et al.,* 1999). The two tertiary structures were highly conserved in their overall folding. The six cysteine residues known to be involved in intra-molecular disulphide bridges within the *A. nodosum* monomer (Weyand *et al.,* 1999) were present at similar positions in *L. digitata* bromoperoxidases. The two cysteine residues involved in the dimer interface of the *A. nodosum* enzyme are also conserved in *L. digitata* vBPOs (Cys-3 and Cys-41, numbering of *A. nodosum* vBPO). The 17 aa-long insertion in the C-terminal part of *L. digitata* bromoperoxidases appeared as an $\alpha$-helix in the 3-D structure model (Fig. 8).

*II.6 Expression of v-BPO1 cDNA in* E. coli

Following overexpression of vBPO1 in *E. coli,* a protein with the expected size of 70 kDa was produced in both the inclusion bodies and cytoplasmic protein fractions. Addition of 3% ethanol increased the proportion of overexpressed protein in the cytoplasmic fraction (Fig. 9A). Consistently, bromoperoxidase activity was higher in the latter fraction than in cytoplasmic protein fractions from cultures induced with 150 mM NaCl only (Fig. 9B). The recombinant bromoperoxidase activity consisted of one split band only, with an apparent molecular mass similar to that of the native protein band referred to as BPOd (Fig. 9B).

**REFERENCES**

Crépineau, F., Roscoe, T., Kaas, R., Kloareg, B., and Boyen, C. (2000) *Plant Mol. Biol.* **43,** 503-513
Weyand, M., Hecht, H.-J., Kiess, M., Liaud, M.-F., Vilter, H., and Schomburg, D. (1999) *J. Mol. Biol.* **293,** 595-611
Vilter, H. (1994) *Methods in Enzymology* **228,** 665-672
Bradford, M. M. (1976) *Anal. Biochem.* **72,** 248-254
Laemmli, U. K. (1970) *Nature* **227,** 680-685
Jordan, P., and Vilter, H. (1990) *Electrophoresis* **11,** 653-655
Hager, L. P., Morris, D. R., Brown, F. S., and Eberwein, H. (1966) *Journal of Biological Chemistry* **241,** 1769-1777
Vilter, H. (1984) *Phytochemistry* **23,** 1387-1390
Hedrick, J. L., and Smith, A. J. (1968) *Archives in Biochemistry and Biophysics* **126,** 155-164
Rabilloud, T., Strub, J.-M., Luche, S., Van Dorsselaer, A., and Lunardi, J. (2001) *Proteomics* **1**, 699-704
Sauber, C., Mandel, F., Van Dorsselaer, A., Schaeffer, C., and Wagner, E. (2002) *Agilent Technologies* **Application Note**
Apt, K. E., Clendennen, S. K., Powers, D. A., and Grossman, A. R. (1995) *Mol Gen Genet* **246,** 455-464
Thomas, J. G., and Baneyx, F. (1997) *Protein Expression and Purification* **11,** 289-296
Küpper, F. C., Schweigert, N., Ar Gall, E., Legendre, J.-M., Vilter, H., and Kloareg, B. (1998) *Planta* **207**, 163-171
O'Dowd, C. D., Jimenez, J. L., Bahreini, R., Flagan, R. C., Seinfeld, J. H., Hämeri, K., Pirjola, L., Kulmala, M., Jennings, G. S., and Hoffmann, T. (2002) *Nature* **417,** 632-636
Borchardt, S. A., Allain, E. J., Michels, J. J., Steams, G. W., Kelly, R. F., and McCoy, W. F. (2001) *Applied and Environnemental Microbiology* **67,** 3174-3179
Potin, P., Bouarab, K., Salaün, J.-P., Pohnert, G., and Kloareg, B. (2002) *Current Opinion in Plant Biology* **5,** 308-317
Butler, A. (1998) *Current Opinion in Chemical Biology* **2,** 279-285
Vilter, H. (1995) in *Metal ions in biological systems* (Sigel, H., and Sigel, A., eds) Vol. 31, pp. 325-362, Marcel Dekker, Inc., New York, Basel, Hong Kong
Butler, A., Carter, J. N., and Simpson, M. T. (2001) in *Handbook on Metalloproteins* (Bertini, I., Sigel, A., and Sigel, H., eds), pp. 153-179, Marcel Dekker, Inc., New York, Basel
Messerschmidt, A., and Wever, R. (1996) *Proceedings of the National Academy* of *Sciences of the U.S.* **93,** 392-396
Shimonishi, M., Kuwamoto, S., Inoue, H., Wever, R., Ohshiro, T., Izumi, Y., and Tanabe, T. (1998) *FEBS letters* **428,** 105-110
Ohshiro, T., Hemrika, W., Aibara, T., Wever, R., and Izumi, Y. (2002) *Phytochemistry* **60**, 595-601
Isupov, M. N., Dalby, A. R., Brindley, A. A., Izumi, Y., Tanabe, T., Murshudov, G. N., and Littlechild, J. A. (2000) *J. Mol. Biol.* **299,** 1035-1049 Carter, J. N., Beatty, K. E., Simpson, M. T., and Butler, A. (2002) *Journal of Inorganic Biochemistry* **91,** 59-69
Vreeland, V., Ng, K. L., and Epstein, L. (1998) *Molecular Biology of the Cell* **9**, 1043
Smith and Waterman (1981) *J. Theor. Biol*., **91(2),** 370-380
Needleman and Wunsch (1972) *J. Mol. Biol*. **48(3),** 443-453
Pearson and Lipman (1988) *PNAS USA* **85(5),** 2444-2448
Wever *et al*. (1991) *Environ. Sci. Technol*. **25,** 446-449

Almeida *et al*. (2001) *Phytochemistry* **57,** 633-642

**Claims**

1. A purified haloperoxidase from *Laminaria digitata.*

2. The purified haloperoxidase according to claim 1, wherein said haloperoxidase is a bromoperoxidase.

3. The purified haloperoxidase according to claim 2, wherein said bromoperoxidase has a sequence selected from SEQ ID No. 1, SEQ ID No. 2, and functional fragments and functional variants thereof.

4. The purified haloperoxidase according to claim 2 or 3, wherein said bromoperoxidase is encoded by a nucleic acid comprising at least one nucleotide sequence selected from SEQ ID No. 4, SEQ ID No. 5, their complementary sequences, and functional fragments and functional variants of SEQ ID No. 4, of SEQ ID No. 5, and of their complementary sequences.

5. The purified haloperoxidase according to claim 2 or 3, wherein said bromoperoxidase is encoded by a nucleic acid comprising at least one nucleotide sequence selected from SEQ ID No. 7, SEQ ID No. 8, their complementary sequences, and functional fragments and functional variants of SEQ ID No. 7, of SEQ ID No. 8, and of their complementary sequences.

6. The purified haloperoxidase according to claim 1, wherein said haloperoxidase is a iodoperoxidase.

7. The purified haloperoxidase according to claim 6, wherein said iodoperoxidase has a sequence selected from SEQ ID No. 3, and functional fragments and functional variants thereof.

8. The purified haloperoxidase according to claim 6 or 7, wherein said iodoperoxidase is encoded by a nucleic acid comprising at least a nucleotide sequence selected from SEQ ID No. 6, its complementary sequence, and functional fragments and functional variants of SEQ ID No. 6 and of its complementary sequence.

9. The purified haloperoxidase according to claim 6 or 7, wherein said iodoperoxidase is encoded by a nucleic acid comprising at least a nucleotide sequence selected from SEQ ID No. 9, its complementary sequence, and functional fragments and functional variants of SEQ ID No. 9 and of its complementary sequence.

10. The purified haloperoxidase according to any of claims 4, 5, 8 and 9, wherein said nucleic acid is contained in a recombinant vector, preferably a recombinant expression vector.

11. The purified haloperoxidase according to claim 10, wherein said recombinant vector is contained in a recombinant host cell.

12. The purified haloperoxidase according to claim 11, wherein said recombinant host cell is from a prokaryotic organism, such as eubacteria, and preferably *Escherichia coli.*

13. The purified haloperoxidase according to claim 11, wherein said recombinant host cell is from a eukaryotic organism, such as a yeast, an insect, heterokonts, oomycetes and brown algae.

14. An isolated nucleic acid encoding a bromoperoxidase from *Laminaria digitata,* comprising a sequence selected from SEQ ID No. 4, SEQ ID No.5, their complementary sequences, and functional fragments and functional variants of SEQ ID No. 4, of SEQ ID No.5, and of their complementary sequences.

**15.** An isolated nucleic acid encoding a bromoperoxidase from *Laminaria digitata,* comprising a sequence selected from SEQ ID No. 7, SEQ ID No.8, their complementary sequences, and functional fragments and functional variants of SEQ ID No. 7, of SEQ ID No.8, and of their complementary sequences.

**16.** An isolated nucleic acid encoding a iodoperoxidase from *Laminaria digitata,* comprising a sequence selected from SEQ ID No. 6, its complementary sequence, and functional fragments and functional variants of SEQ ID No. 6 and of its complementary sequence.

**17.** An isolated nucleic acid encoding a iodoperoxidase from *Laminaria digitata*, comprising a sequence selected from SEQ ID No. 9, its complementary sequence, and functional fragments and functional variants of SEQ ID No. 9 and of its complementary sequence.

**18.** A recombinant vector comprising at least one isolated nucleic acid according to any of claims 14 to 17.

**19.** The recombinant vector according to claim 18, wherein said vector enables said isolated nucleic acid to be expressed.

**20.** A recombinant host cell comprising at least one recombinant vector according to claim 18 or 19.

**21.** The recombinant host cell according to claim 20, wherein said cell is from a prokaryotic organism, such as eubacteria, and preferably *Escherichia coli.*

**22.** The recombinant host cell according to claim 20, wherein said cell is from a eukaryotic organism, such as a yeast, an insect, heterokonts, oomycetes and brown algae.

**23.** A bromoperoxidase from *Laminaria digitata* encoded by an isolated nucleic acid according to claim 14 or 15.

**24.** A bromoperoxidase from *Laminaria digitata* having a sequence selected from SEQ ID No. 1, SEQ ID No.2, and functional fragments and functional variants thereof.

**25.** A iodoperoxidase from *Laminaria digitata* encoded by an isolated nucleic acid according to claim 16 or 17.

**26.** A iodoperoxidase from *Laminaria digitata* having a sequence selected from SEQ ID No. 3, and functional fragments and functional variants thereof.

**27.** A method for preparing a purified haloperoxidase according to any of claims 2 to 5, comprising at least:

a) cloning at least one nucleic acid according to claim 4 or 5, into a recombinant expression vector;
b) transforming a recombinant host cell with said recombinant expression vector;
c) expressing said at least one nucleic acid from said recombinant host cell; and
d) purifying said bromoperoxidase.

**28.** A method for preparing a purified haloperoxidase according to any of claims 6 to 9, comprising at least:

a) cloning at least one nucleic acid according to claim 8 or 9, into a recombinant expression vector ;
b) transforming a recombinant host cell with said recombinant expression vector;
c) expressing said at least one nucleic acid from said recombinant host cell; and
d) purifying said iodoperoxidase.

**29.** A method for preparing a bromoperoxidase from *Laminaria digitata* according to claim 23 or 24, comprising at least:

a) cloning at least one nucleic acid according to claim 14 or 15, into a recombinant expression vector;
b) transforming a recombinant host cell with said recombinant expression vector ; and
c) expressing said at least one nucleic acid from said recombinant host cell.

**30.** A method for preparing a iodoperoxidase from *Laminaria digitata* according to claim 25 or 26, comprising at least:

a) cloning at least one nucleic acid according to claim 16 or 17, into a recombinant expression vector;

b) transforming a recombinant host cell with said recombinant expression vector; and
c) expressing said at least one nucleic acid from said recombinant host cell.

**31.** The method according to claim 29 or 30, further comprising purifying said bromoperoxidase or said iodoperoxidase.

**32.** The method according to any of claims 27 to 31, wherein said recombinant host cell is a cell from a prokaryotic organism, such as eubacteria, and preferably *Escherichia coli.*

**33.** The method according to any of claims 27 to 31, wherein said recombinant host cell is a cell from a eukaryotic organism, such as a yeast, an insect, heterokonts, oomycetes and brown algae.

**34.** Use of at least one purified haloperoxidase according to any of claims 1 to 13 and/or at least one bromoperoxidase according to claim 23 or 24 and/or at least one iodoperoxidase according to claim 25 or 26, for halogenating organic compounds, such as pesticides, drugs, adhesives.

**35.** Use of at least one purified haloperoxidase according to any of claims 1 to 13 and/or at least one bromoperoxidase according to claim 23 or 24 and/or at least one iodoperoxidase according to claim 25 or 26, for trapping halogens, preferably during water treatment.

**36.** Use of at least one purified haloperoxidase according to any of claims 1 to 13 and/or at least one bromoperoxidase according to claim 23 or 24 and/or at least one iodoperoxidase according to claim 25 or 26, for *in situ* producing antibiotic compounds.

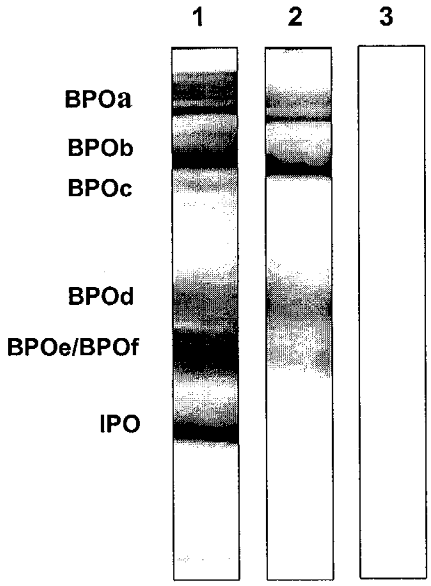


FIGURE 1.

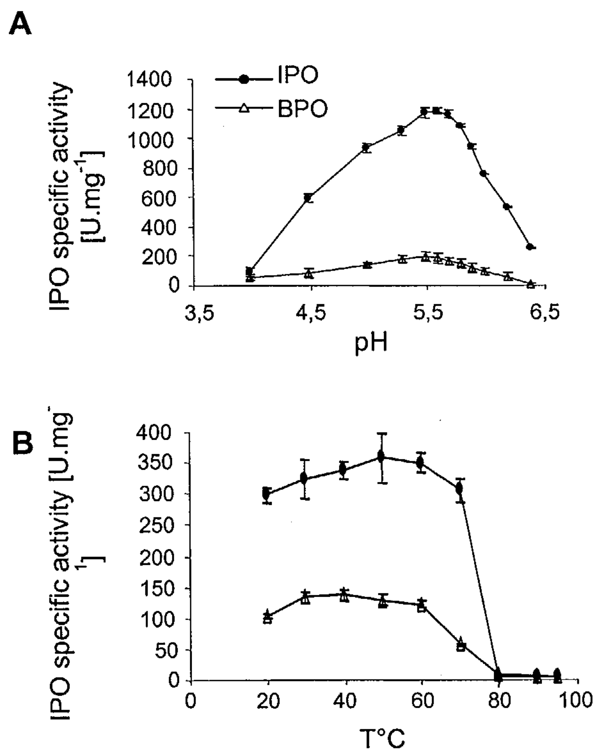


FIGURE 3.

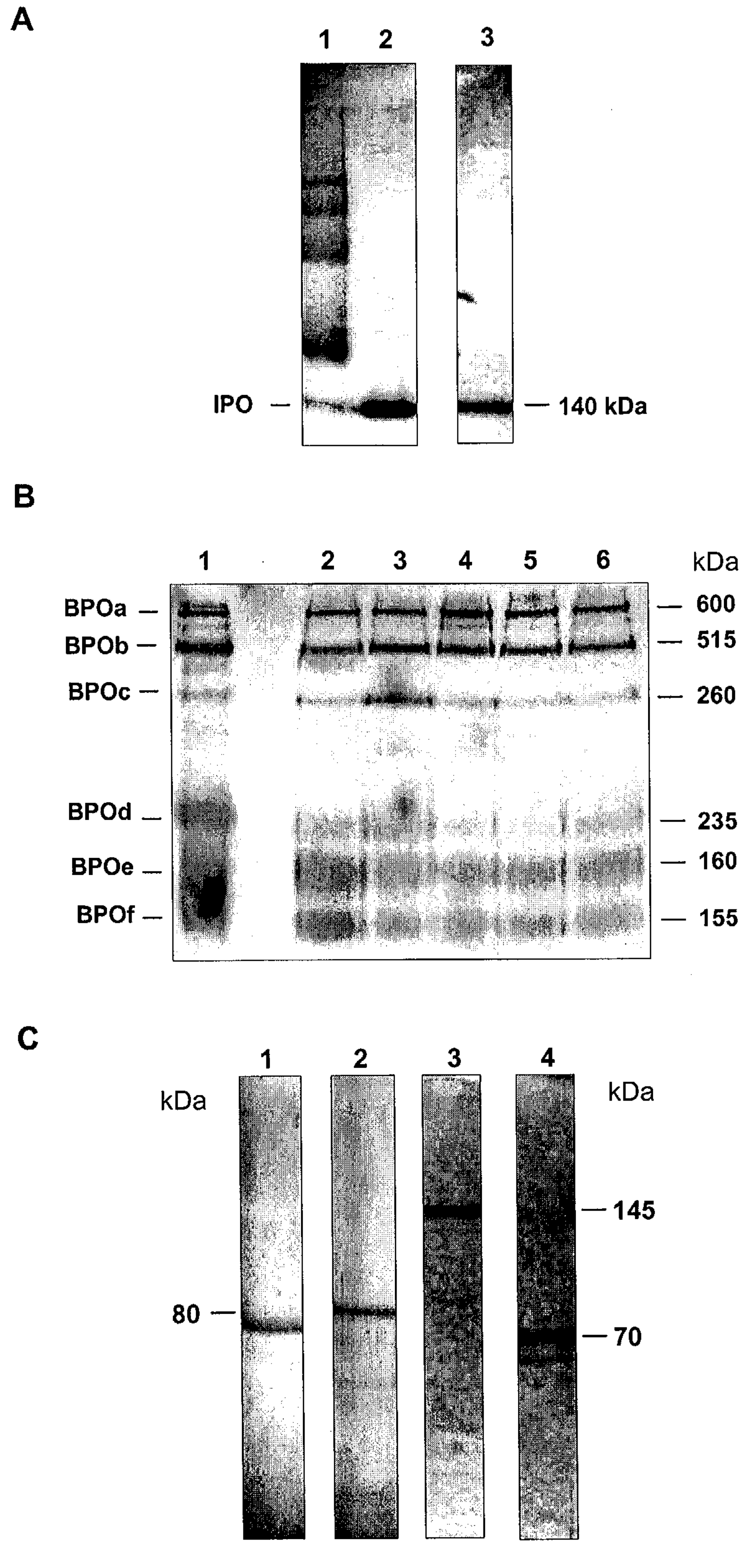
A
1
2
3
IPO
140 kDa
B
1
2
3
4
5
6
kDa
BPOa
BPOb
BPOc
BPOd
BPOe
BPOf
600
515
260
235
160
155
C
1
2
3
4
kDa
kDa
80
145
70

FIGURE 2.

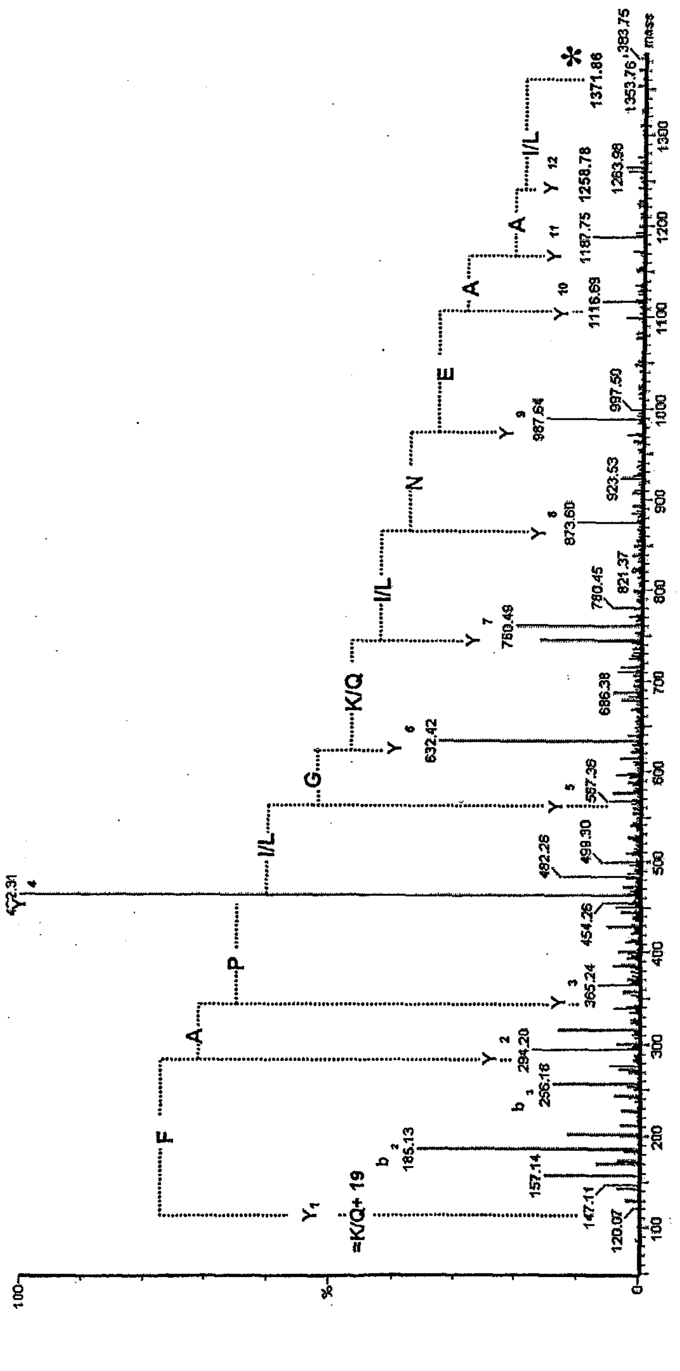
Y1
=K/Q+ 19
F
A
P
I/L
G
K/Q
I/L
N
E
A
A
I/L
*
b2
b3
Y2
Y3
Y4
Y5
Y6
Y7
Y8
Y9
Y10
Y11
Y12
120.07
147.11
157.14
185.13
256.16
294.20
365.24
454.26
472.31
482.28
499.30
587.39
632.42
686.38
760.49
790.45
821.37
873.60
923.53
987.54
997.50
1116.69
1187.75
1258.78
1283.98
1353.76
1371.86
1383.75
mass
100
%
0
100
200
300
400
500
600
700
800
900
1000
1100
1200
1300

FIGURE 4

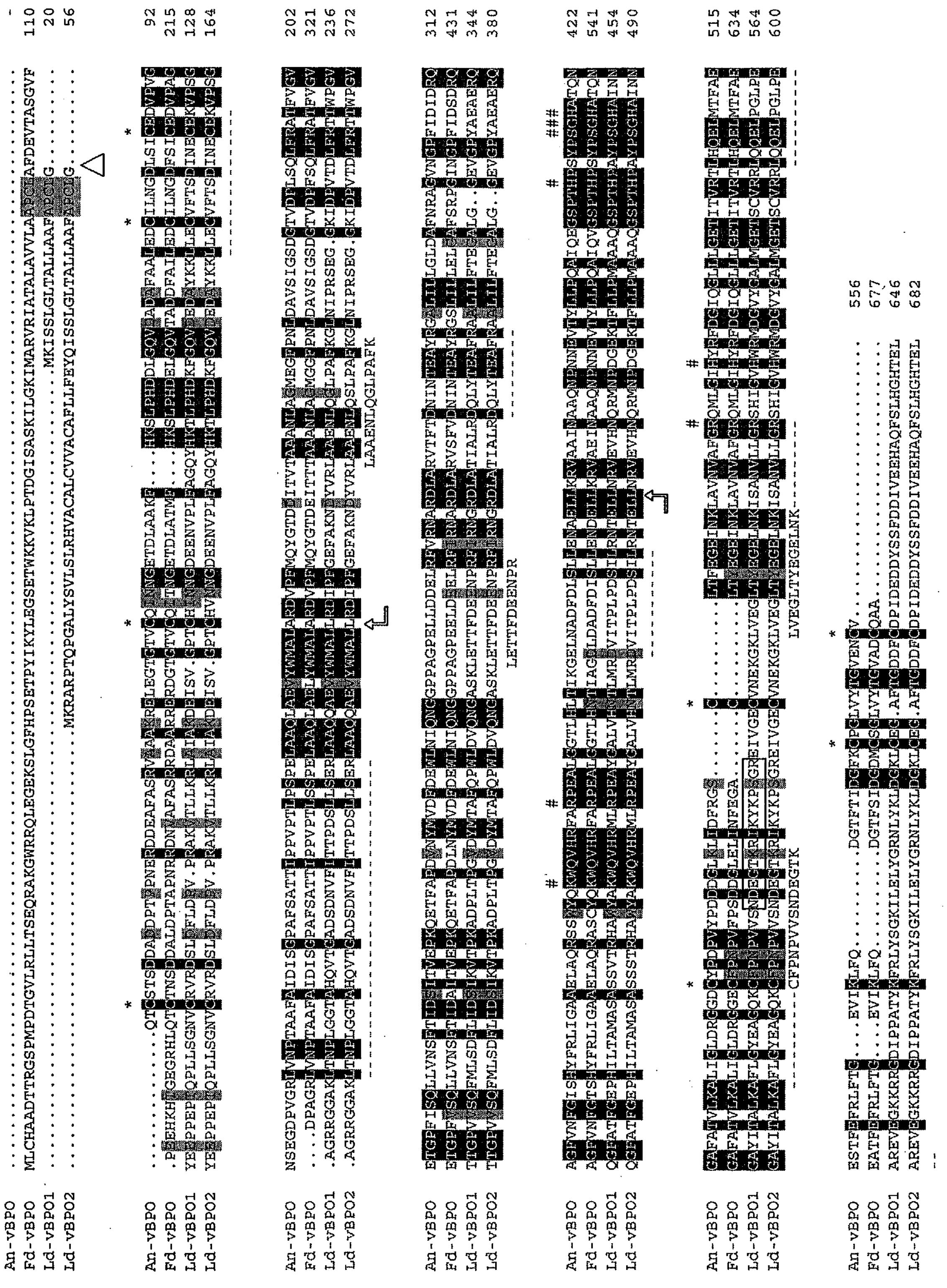

FIGURE 5.

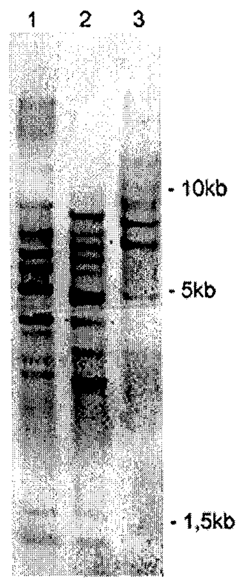


FIGURE 6.

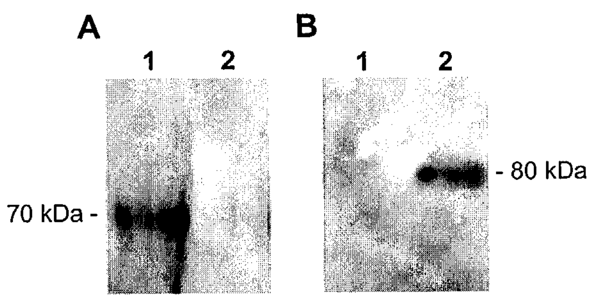


FIGURE 7.

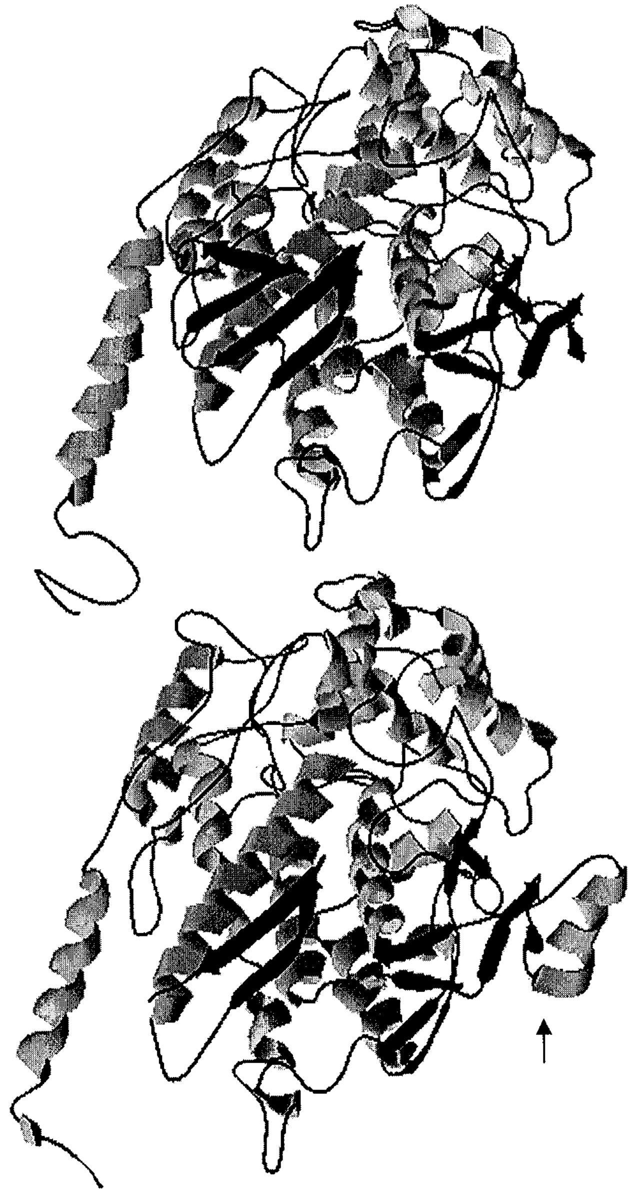

A

B

FIGURE8.

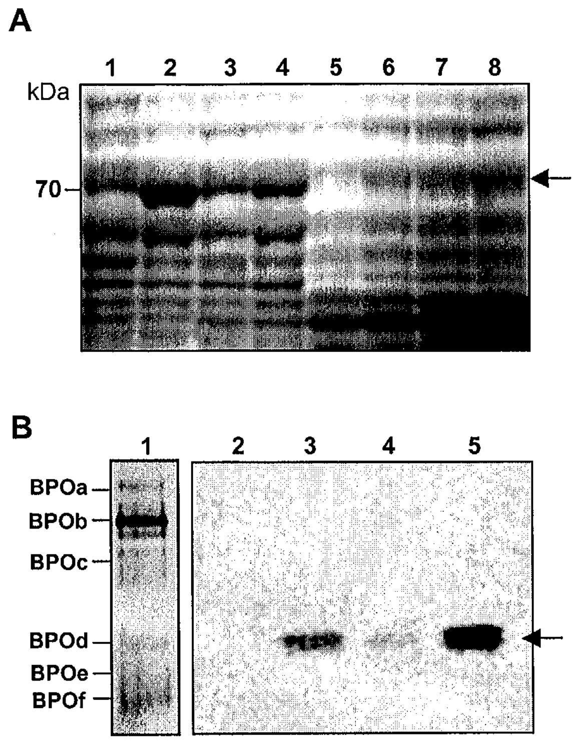


FIGURE 9.

European Patent Office

# EUROPEAN SEARCH REPORT

Application Number
EP 03 29 0518

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | JORDAN, P. & VILTER, H.: "Extraction of proteins from material rich in anionic mucilages: Partition and fractionation of vanandate-dependent bromoperoxidases from the brown algae Laminaria digitata and L. saccharina in aqueous polymer two-phase systems" BIOCHIMICA ET BIOPHYSICA ACTA, vol. 1073, no. 1, 23 January 1991 (1991-01-23), pages 98-106, XP000921187 | 1,2, 34-36 | C12N15/29 C12N15/53 C12N9/08 |
| A | * the whole document * see especially: * page 102, column 2, line 8 - page 103, column 2, line 18 * and * page 104; table IIA * --- | 3-33 | |
| D,X | DATABASE EMBL, HEIDELBERG, FRG 'Online! 8 February 2000 (2000-02-08) CRÉPINEAU, F. ET AL.: "LamdiSest169est L. digitata sporophyte Lambda ZapII Laminaria digitata cDNA similar to vanadium bromoperoxidase, mRNA sequence" Database accession no. AW400475 XP002253089 * the whole document * --- | 3-5, 10-15, 18-24, 27,29, 31-33 | TECHNICAL FIELDS SEARCHED (Int.Cl.7) C12N |
| X | DATABASE EMBL, HEIDELBEG, FRG 'Online! 8 February 2000 (2000-02-08) CRÉPINEAU, F. ET AL.: "LamdiSest129est L. digitata sporophyte Lambda ZapII Laminaria digitata cDNA similar to vanadium bromoperoxidase, mRNA sequence" Database accession no. AW400432 XP002253090 * the whole document * --- -/-- | 3-5, 10-15, 18-24, 27,29, 31-33 | |

The present search report has been drawn up for all claims

| Place of search | Date of completion of the search | Examiner |
|---|---|---|
| BERLIN | 27 November 2003 | Fuchs, U |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 03 29 0518

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.7) |
| D,A | CRÉPINEAU, F. ET AL.: "Characterisation of complementary DNAs from the expressed sequence tag analysis of life cycle stages of Laminaria digitata (Phaeophyceae)" PLANT MOLECULAR BIOLOGY, vol. 43, no. 4, July 2000 (2000-07), pages 503-513, XP002253087 * the whole document * see especially: * page 508; table 2 * and * page 511, column 2, line 20 - line 43 * | 1-36 | |
| A | WO 02 00838 A (REGENTS OF THE UNIVERSITY OF CALIFORNIA) 3 January 2002 (2002-01-03) * the whole document * | 1-36 | |
| D,A | VILTER, H.: "Extraction of Proteins from Sources Containing Tannins and Anionic Mucilages" METHODS IN ENZYMOLOGY, vol. 228, 1994, pages 665-672, XP008021382 * the whole document * | 1-36 | TECHNICAL FIELDS SEARCHED (Int.Cl.7) |
| T | COLIN, C. ET AL.: "The Brown Algal Kelp Laminaria digitata Features Distinct Bromoperoxidase and Iodoperoxidase Activities" JOURNAL OF BIOLOGICAL CHEMISTRY, vol. 278, no. 26, 27 June 2003 (2003-06-27), pages 23545-23552, XP002253088 * the whole document * | 1-36 | |

The present search report has been drawn up for all claims

| Place of search | Date of completion of the search | Examiner |
|---|---|---|
| BERLIN | 27 November 2003 | Fuchs, U |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

Application Number

EP 03 29 0518

## CLAIMS INCURRING FEES

The present European patent application comprised at the time of filing more than ten claims.

☐ *Only part of the claims have been paid within the prescribed time limit. The present European search* report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

European Patent Office

**LACK OF UNITY OF INVENTION SHEET B**

Application Number
EP 03 29 0518

The Search Division considers that the present European patent application does not comply with the requirements of unity of invention and relates to several inventions or groups of inventions, namely:

1. Claims: 1, 10-13, 18-22, 31-36 (all partially) and 2-5, 14, 15, 23, 24, 27, 29 (all completely)

A purified bromoperoxidase from Laminaria digitata, said bromoperoxidase having an amino acid sequence selected from SEQ ID NO: 1 or SEQ ID NO: 2, said bromoperoxidase being encoded by a nuleic acid comprising a nucleotide sequence selected from SEQ ID NO: 4 or SEQ ID NO: 5, said bromoperoxidase being encoded by a nucleic acid comprising a nucleotide sequence selected from SEQ ID NO: 7 or SEQ ID NO: 8, said bromoperoxidase wherein said nucleic acid is contained in a recombinant vector, said bromoperoxidase wherein said recombinant vector is contained in a recombinant host cell, an isolated nucleic acid encoding a bromoperoxidase from Laminara digitata comprising a sequence selected from SEQ ID NO: 4 or SEQ ID NO: 5, an isolated nucleic acid encoding a bromoperoxidase from Laminaria digitata comprising a sequence selected from SEQ ID NO: 7 or SEQ ID NO: 8, a recombinant vector comprising said isolated nucleic acid, a recombinant host cell comprising said recombinant vector, a bromoperoxidase from Laminaria digitata being encoded by said isolated nucleid acid, a bromoperoxidase from Laminaria digitata having a sequence selected from SE ID NO: 1 or SEQ ID NO: 2, a method for preparing said purified bromoperoxidase involving said nucleic acids, use of said bromoperoxidase for halogenating organic compounds, use of said bromoperoxidase for trapping halogens, use of said bromoperoxidase for in situ producing antibiotic compounds;

2. Claims: 1, 10-13, 18-22, 31-36 (all partially) and 6-9, 16, 17, 25, 26, 28, 30 (all completely)

idem as subject 1 but limited to a iodoperoxidase from Laminaria digitata and SEQ ID NOS: 3, 6 and 9.

## ANNEX TO THE EUROPEAN SEARCH REPORT ON EUROPEAN PATENT APPLICATION NO. EP 03 29 0518

This annex lists the patent family membersrelating to the patent documents cited in the above-mentioned European search report.
The members are as contained in the European Patent Office EDP file on
The European Patent Office is in no way liable for these particulars which are merely given for the purpose of information.

27-11-2003

| Patent document cited in search report | Publication date | Patent family member(s) | Publication date |
|---|---|---|---|
| WO 0200838 A | 03-01-2002 | AU 7540401 A | 08-01-2002 |
| | | WO 0200838 A2 | 03-01-2002 |

EPO FORM P0459

For more details about this annex : see Official Journal of the European Patent Office, No. 12/82